(19) Europäisches Patentamt
European Patent Office
Office européen des brevets

(11) **EP 2 627 368 B1**

(12) **EUROPÄISCHE PATENTSCHRIFT**

(45) Veröffentlichungstag und Bekanntmachung des Hinweises auf die Patenterteilung:
**14.09.2016   Patentblatt 2016/37**

(21) Anmeldenummer: **11817299.8**

(22) Anmeldetag: **14.10.2011**

(51) Int Cl.:
***A61M 1/16*** *(2006.01)*

(86) Internationale Anmeldenummer:
**PCT/DE2011/001847**

(87) Internationale Veröffentlichungsnummer:
**WO 2012/051996 (26.04.2012 Gazette 2012/17)**

(54) **VERFAHREN UND VORRICHTUNG ZUR MESSUNG UND BEHEBUNG VON SYSTEMÄNDERUNGEN IN EINER VORRICHTUNG ZUR BEHANDLUNG VON BLUT**

METHOD AND DEVICE FOR THE MEASUREMENT AND THE ELIMINATION OF SYSTEM CHANGES IN A DEVICE FOR THE TREATMENT OF BLOOD

PROCÉDÉ ET DISPOSITIF POUR MESURER ET ÉLIMINER DES VARIATIONS DU SYSTÈME DANS UN DISPOSITIF DE TRAITEMENT DU SANG

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **14.10.2010   DE 102010048771**

(43) Veröffentlichungstag der Anmeldung:
**21.08.2013   Patentblatt 2013/34**

(73) Patentinhaber: **B. Braun Avitum AG**
**34212 Melsungen (DE)**

(72) Erfinder: **WOLFF, Henrik**
**37213 Witzenhausen (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**EP-A1- 1 364 666      WO-A1-2008/135193**
**US-A- 5 476 592**

**Beschreibung**

Die vorliegende Erfindung betrifft ein Verfahren zur Erkennung von Systemänderungen in einer Vorrichtung zur Behandlung von Blut und eine Vorrichtung zur Behandlung von Blut.

Die Erfindung bezieht sich dabei auf das Feld der Filtration, genauer gesagt der Tangentialflussfiltration (TFF). Hier ist die zu reinigende Flüssigkeit durch eine semipermeable Membran von der Reinigungslösung getrennt. Typischerweise weist die Reinigungslösung eine niedrigere Konzentration an Stoffen auf, als aus der zu reinigenden Flüssigkeit entfernt werden sollen. Hierdurch wird Diffusion erzeugt. Um die Diffusion als reinigende Kraft optimal nutzen zu können, werden Tangentialflussfilter typischerweise im Gegenstromprinzip betrieben.

Ein Dialysefilter besteht im Wesentlichen aus Hohlfasern, also zylinderförmigen Fasern, die länglich gestreckt ein Gehäuse durchziehen. Die Wände der Hohlfasern wirken dabei durch teildurchlässige (semipermeable) Strukturen als Membranen. An ihren Enden sind die Hohlfasern in eine Vergussmasse eingebettet. Im Dialysefilter können die Hohlfasern zu Modulen mit mehreren Quadratmetern Filterfläche zusammengefasst werden. Bei der Dialyse durch Tangentialflussfiltration, auch Querstromfiltration oder Cross-Flow-Filtration genannt, wird den Hohlfasern über einen ersten Flüssigkeitskreislauf Blut/Plasma zugeführt, das sie der Länge nach durchströmt. Durch einen zweiten Flüssigkeitskreislauf wird die Dialyseflüssigkeit, in der Regel im Gegenstromprinzip, aber ggf. auch parallel zum Blutstrom, zugeführt. Das Gehäuse weist also vier Anschlüsse auf, und zwar für jeden Flüssigkeitsstrom zwei, jeweils einen zur Zufuhr und zur Abfuhr. An der Innenseite der Hohlfasermembran befindet sich also der Blutstrom, und auf der Außenseite die Dialyseflüssigkeit.

Ein weiterer Reinigungsmechanismus ist die Konvektion. Hier wird ein Druckgradient über die semipermeable Membran erzeugt, wodurch die zu reinigende Flüssigkeit über die semipermeable Membran gedrückt wird. Dadurch werden die Substanzen in ihrer vorliegenden Konzentration mit gespült. Dieser Reinigungsprozess ist also nicht von der Konzentration an Substanzen in der Reinigungslösung abhängig, entscheidend sind hier nur die Konzentration in der zu reinigenden Flüssigkeit und die Membraneigenschaften wie Siebkoeffizient, Permeabilität usw.. Es ist also von Interesse, die Filtereigenschaften zu Behandlungsbeginn sowie während der Behandlung zu kennen.

Ein spezieller Bereich der Filtration ist die extrakorporale Blutbehandlung bei chronischer oder akuter Niereninsuffizienz. Hier ist die zu reinigende Flüssigkeit das Blut des Patienten und die Reinigungslösung ist die Dialyseflüssigkeit. Entscheidend in diesem speziellen Fall der TFF ist es, in den Behandlungen (im chronischen Fall typischerweise drei pro Woche) die Blutreinigungsfunktion der Niere möglichst effektiv zu ersetzen. Um dies zu gewährleisten, hat sich der Kt/V-Wert als Maß für die Behandlungsqualität durchgesetzt. Der Kt/V ist ein Parameter, um die Dialyseeffektivität zu bestimmen und ein wesentlicher Bestandteil zur Beurteilung der Dialyseeffizienz. K ist die Clearance, die über den Harnstoffgehalt des Blutes vor und nach der Dialyse ermittelt wird. Der Wert t zeigt die effektive Dialysezeit in Minuten und der Wert V das Harnstoffverteilungsvolumen. Dieses bezieht sich auf 60% der Körpermasse (Gewicht), in der das Blut zirkulieren kann (Körperwassergehalt). Ziel einer Behandlung ist es, einen $Kt/V \geq 1{,}2$ zu erzielen. In einem normalen Behandlungsverlauf werden Werte erzielt, die dieses Kriterium in der Regel erfüllen. Allerdings können in einer Behandlung Widrigkeiten auftreten, die sich negativ auf den Behandlungsverlauf sowie das Behandlungsergebnis auswirken. Es ist somit wichtig, während einer Behandlung die Einflussparameter zu überwachen und zu kontrollieren, um auf solche Widrigkeiten schnell und vor allem gezielt reagieren zu können und die Systemparameter während der Dialyse entsprechend anzupassen.

Ein entscheidender Prozess ist die Interaktion der Filtermembran mit Blut. Durch diese Wechselwirkung verschlechtern sich die Flusseigenschaften des Filters sowohl in Transmembranrichtung als auch in Blutflussrichtung. Diese Veränderungen werden beispielsweise durch Thrombozytenanlagerung auf die Membran, Koagelbildung, chemisches Binden von Blutbestandteilen an die Membran oder schlicht mechanisches (strömungsbedingtes) Drücken der Blutbestandteile an und sogar in die Membran verursacht.

Transmembranrichtung oder transmembranös bezieht sich hierbei auf einen Fluss des Blutes über die Membran des Dialysators bzw. Dialysefilters.

Bei der Koagelbildung entsteht ein gallertartiges durch Fibrinfäden stabilisiertes Aggregat aus roten Blutkörperchen (Erythrozyten). Anders als der Begriff Thrombus beschreibt ein Koagulum ein Blutgerinnsel, welches sich außerhalb eines Blut- oder Lymphgefäßes (extravasal) befindet und nicht innerhalb (intravasal).

Diese und weitere Änderungen der Systemeigenschaften haben diverse Auswirkungen auf den Behandlungsverlauf und die Behandlungsqualität. Besonders die Behandlung durch Hämodiafiltration wird dadurch beeinträchtigt, da hier auf den konvektiven Stofftransport mittelmolekularer Substanzen gesetzt wird. Durch Verschlechterung der transmembranen Flusseigenschaften oder der Permeabilität, verschlechtert sich auch der Siebkoeffizient für die urämischen Substanzen im mittelmolekularen Bereich, was dazu führt, dass bei gleicher Menge konvektiv gefilterter Flüssigkeit weniger urämische Substanzen aus dem Blutkreislauf entfernt werden. Ein anderer Effekt ist die Verringerung der effektiven Durchflussfläche sowohl in Blutflussrichtung als auch in Transmembranrichtung. Dies hat eine Reduzierung der aktiven Filteroberfläche zur Folge, wodurch es zu einer Verschlechterung der diffusiven Reinigung kommen kann. Bei neuwertigen Filtern besteht in der Regel ein Pufferpotential, das größer ist als die maximale physiologische Filterung.

Dadurch kann eine Verringerung der effektiven Durchflussfläche in einem bestimmten Maß abgefangen werden. Ist dieses Potential jedoch ausgeschöpft, kommt es zu dem zuvor beschriebenen Effekt.

Als geeignete Gegenmaßnahmen oder Reaktionen auf solche Änderungen gelten allgemein das Spülen mit Kochsalzlösung zum "Säubern" des Dialysators, die Zugabe von Heparin, um weitere Koagelbildung zu verhindern oder das Senken der Ultrafiltrationsrate (UF), um die Hämokonzentration zu reduzieren. Die Permeabilität der Membranen wird bestimmt durch Messung des Flüssigkeitsvolumens, das bei gegebener Druckdifferenz bei einer Temperatur von 37°C durch eine festgelegte Membranfläche durch die Membran tritt und das zur allgemeinen Vergleichbarkeit auf Flächeneinheit, Zeiteinheit und Druckeinheit normiert wird. Als Flüssigkeit zur Bestimmung der Ultrafiltrationsrate wird Wasser verwendet.

Aus dem Stand der Technik sind bereits Versuche bekannt, die das Ziel haben Systemänderungen zu erkennen und darauf zu reagieren. Siehe z.B. EP 1 364 666. In der US 2008/0215247 A1 wird davon ausgegangen, dass der lineare Zusammenhang zwischen Transmembrandruck (TMP) und Ultrafiltrationsrate $Q_{UF}$ = TMP * $K_{UF}$ ($K_{UF}$ = Ultrafiltrationskoeffizient) nur in einem gewissen Bereich des TMPs erfüllt ist. Somit wird zunächst die Funktion $Q_{UF}$(TMP) abgeschätzt, indem der TMP sukzessive erhöht und die dadurch erzeugte Ultrafiltrationsrate gemessen wird. Ab einem bestimmten Wert hat eine Erhöhung des TMP eine immer geringere Steigerung der Ultrafiltrationsrate zur Folge. Man wählt daher den Kniepunkt (Tangentialpunkt) der Funktion $Q_{UF}$(TMP) als Arbeitspunkt. Da sich der $K_{UF}$ während der Behandlung auf Grund der Systemänderung verschlechtert, offenbart die WO 2006/011009 A2 die technischen Voraussetzungen, den Zusammenhang zwischen TMP und $Q_{UF}$ stündlich neu ermitteln zu lassen.

In der EP 1175917 A1 wird als weiteres Konzept das Anpassen der Verhältnisse aus Prä- und Postdilution im Verlaufe der Behandlung beschrieben.

In der US 2006/157408 A1 wird eine Methode zur Detektion von Filterverstopfungen offenbart. Um solche Filterverstopfungen zu detektieren, können bis zu vier verschiedene Drucksensoren eingesetzt werden, welche an bis zu vier verschiedenen Stellen Druckmessungen vornehmen. Dabei wird bei Druckänderungen an einem der Drucksensoren darauf geschlossen, dass eine Filterverstopfung vorliegt, womit durch Zugabe von Heparin reagiert werden kann. Allerdings wurde nicht erkannt, dass eine Vielzahl von Konstellationen existieren, in denen sich die Drücke im System ändern können, ohne dass es tatsächlich zu einer Filterverstopfung gekommen wäre. Weiterhin wurde nicht erkannt, dass sich diese Konstellationen erst durch die Erstellung von zeitlichen Trends aus den gemessenen Drucksignalen und allen Quotienten oder Differenzen der gemessenen Drucksignale erkennen lassen. In der vorliegenden Erfindung hat sich herausgestellt, dass Druckunterschiede nicht immer auf eine Filterverstopfung zurückzuführen sind, sondern eine Vielzahl von Ursachen haben können, die erfindungsgemäß detektiert und lokal eingegrenzt werden können. Somit offenbart die US 2006/157408 A1 zwar, dass an bis zu vier Stellen Druckmessungen vorgenommen werden können, um die Sicherheit der getroffenen Ja-Nein-Aussage zu erhöhen, aber die Drücke werden nicht miteinander ins Verhältnis gesetzt und Trends beobachtet, um dadurch verschiedenste Störungsmuster zu detektieren und lokal einzugrenzen. Damit dienen die vier Messpunkte nur dazu, die Aussage zu treffen, ob der Filter zugesetzt ist oder nicht (Ja-Nein-Aussage), was auch nur mit der Messung an einem Messpunkt festzustellen gewesen wäre. D.h. die Messung, welche in der US 2006/157408 A1 an bis zu vier Messpunkten durchgeführt wird, dient nicht dazu, weitere Informationen zu erhalten, sondern einzig und allein dazu, die Messsicherheit zu erhöhen, weil z.B. bei der Messung an nur einem Punkt auch der Messsensor defekt sein könnte, was durch die Messung bei mehreren Punkten erkannt werden könnte. Erfindungsgemäß dient die Messung an den vier Messpunkten aber nicht zur Erhöhung der Messsicherheit, sondern dazu, weitere Informationen zu erhalten, um die Fehlerquelle lokal einzugrenzen und zusätzlich die Störung qualitativ zu beschreiben.

Weiterhin wird in der DE 10355042 B3 eine Methode offenbart, mit der Störungen im Blutfluss detektiert werden können, in dem der Phasenwinkel mindestens einer Oberschwingung eines sich im extrakorporalen Blutkreislauf fortpflanzenden oszillierenden Drucksignals ermittelt wird. Auch mit dieser Methode lassen sich Störungen im System nicht genau detektieren und lokal eingrenzen und als einzige Maßnahme zur Behebung einer Störung wird lediglich die Steuerung der Ultrafiltrationsrate angegeben.

Ziel dieser Erfindung ist es somit, ein Verfahren zur Erkennung von Systemänderungen in einer Vorrichtung zur Behandlung von Blut und eine Vorrichtung zur Behandlung von Blut geeignet zur Messung dieser Systemänderungen in der Vorrichtung zur Behandlung von Blut bereitzustellen, welche nicht nur Systemänderungen identifizieren kann, sondern zusätzlich verlässlich zwischen Systemänderungen in Blutflussrichtung, Dialysatflussrichtung und Transmembranrichtung unterscheiden kann, um ein gezieltes Handeln zu ermöglichen.

Diese Aufgabe wird erfindungsgemäß durch die Verfahren und die Vorrichtungen, die in den unabhängigen Ansprüchen genannt sind, gelöst. Weitere vorteilhafte Ausgestaltungen der Erfindung ergeben sich aus den abhängigen Ansprüchen, der Beschreibung, den Figuren sowie den Beispielen.

Verfahren zur Unterscheidung von Störungen des Flusswiderstandes in einem Blutbehandlungssystem, das die folgenden Schritte umfasst:

Das erfindungsgemäße Verfahren zur Erkennung von Systemänderungen und insbesondere zur Unterscheidung von Störungen des Flusswiderstandes in einem Blutbehandlungssystem umfasst die folgenden Schritte,

a) Messung von mindestens zwei Drucksignalen ausgewählt aus der Gruppe bestehend aus (PB1, PB2, PD1, PD2), die an mindestens zwei Drucksensoren gemessen werden, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;

b) Berechnung der Quotienten und/oder der Differenzen aus den gemäß Schritt a) gemessen Drucksignalen, wobei die Quotienten und/oder Differenzen berechnet werden aus den Drucksignalen ausgewählt aus der Gruppe bestehend aus (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM) in einer zentralen Recheneinheit, wobei mit PBM oder PDM der jeweilige Mittelwert aus (PB1, PB2) bzw. (PD1, PD2) bezeichnet wird;

c) Mindestens einmaliges zeitversetztes Wiederholen der Schritte a) und b), wobei an denselben mindestens zwei Drucksensoren wie in der vorangegangenen Messung gemessen wird und aus den gemessenen Drucksignalen dieselben Quotienten und/oder Differenzen berechnet werden;

d) Erstellen mindestens eines zeitlichen Trends aus den gemessenen Drucksignalen ausgewählt aus der Gruppe bestehend aus (PB1, PB2, PD1, PD2) und/oder den berechneten Differenzen und/oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM);

e) Bewerten des mindestens einen zeitlichen Trends, ob eine Änderung des mindestens einen zeitlichen Trends über einen Toleranzbereich hinaus eingetreten ist;

f) Erstellen eines Musters für die Bewertung des mindestens einen zeitlichen Trends;

g) Zuordnung des Musters zu einem Störungsbild und

h) Anzeigen der Störung auf einer Anzeigevorrichtung der zentralen Recheneinheit.

Bevorzugter Weise kann durch das erfindungsgemäße Verfahren sowohl eine Aussage darüber getroffen werden, ob sich die Flusseigenschaften in Blutflussrichtung, in Dialysatflussrichtung als auch in Transmembranrichtung verändert haben. Bisherige Systeme sind nicht in der Lage, solch eine Differenzierung zu treffen. Weiterhin bevorzugt können durch das erfindungsgemäße Verfahren Störungen nicht nur lokal eingegrenzt werden, sondern es ist auch in vielen Fällen möglich, die Störungsursache qualitativ zu erfassen. Dies bietet den Vorteil, dass durch Auswertung der mindestens zwei Drucksignale die Art und der Ort der Veränderung in der Vorrichtung zur Behandlung von Blut genau bestimmt werden kann, um anschließend eine gezielte Behebung der Ursache für die Veränderung durchzuführen. Eine aufwendige und zeitintensive Suche nach dem genauen der Veränderung zugrunde liegenden Problem entfällt. Allgemeine Maßnahmen, wie das Spülen der gesamten Vorrichtung zur Behandlung von Blut können durch gezielte Maßnahmen ersetzt werden, indem z.B. nur das Schlauchsystem oder nur der Filter ausgetauscht wird, in dem eine Systemänderung, z.B. in Form einer Koagelbildung, stattgefunden hat.

Optional kann dieses Verfahren zusätzlich den folgenden Schritt umfassen:

i) Behebung der Störung des Flusswiderstandes in transmembraner Richtung, Dialysatflussrichtung oder Blutflussrichtung oder Angabe einer Möglichkeit zur Behebung der Störung.

Die Behebung der Störung kann, sofern es sich nicht um das Auswechseln von Systemkomponenten wie Dialysator oder Schlauchsystem handelt, automatisch, halbautomatisch oder manuell erfolgen. Ferner können das Anzeigen der Bewertung gemäß Schritt h) und die Angabe einer Möglichkeit zur Behebung der Störung gemäß Schritt i) zeitgleich oder unmittelbar nacheinander erfolgen oder beide Schritte können auch identisch sein. Sollte die Störung nur durch vollständigen Austausch einer Systemkomponente zu beheben sein, erfolgt das Auswechseln des Dialysators manuell, oder falls eine automatische Störungsbehebung gewünscht sein sollte, wird der Blutfluss und optional auch der Dialysatfluss zu einer am oder im Dialysegerät befindlichen Ersatzkomponente umgeleitet.

In einer weiter bevorzugten Ausführungsform wird der zeitliche Trend aus den gemessenen Drucksignalen und den berechneten Differenzen und/oder Quotienten der Drucksignale erstellt, oder nur mit den berechneten Differenzen und/oder Quotienten. In dieser Ausführungsform wird Schritt d) durch den folgenden Schritt d') ersetzt:

d') Erstellen mindestens eines zeitlichen Trends aus den gemessenen Drucksignalen ausgewählt aus der Gruppe bestehend aus (PB1, PB2, PD1, PD2) und den berechneten Differenzen und/oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM), oder

den berechneten Differenzen und/oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM).

Der Schritt d') gibt dasselbe wie Schritt d) nur in einer ausführlicheren Schreibweise wieder.

In einer weiteren Ausführungsform umfasst das erfindungsgemäße Verfahren die Messung von mindestens drei Drucksignalen und damit die Folgenden Schritte:

a) Messung von mindestens drei Drucksignalen ausgewählt aus der Gruppe bestehend aus (PB1, PB2, PD1, PD2), die an mindestens drei Drucksensoren gemessen werden, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;

b) Berechnung der Quotienten und/oder der Differenzen aus den gemäß Schritt a) gemessen Drucksignalen, wobei die Quotienten und/oder Differenzen berechnet werden aus den Drucksignalen ausgewählt aus der Gruppe bestehend aus (PB1, PB2) und/oder (PB1. PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM) in einer zentralen Recheneinheit, wobei mit PBM oder PDM der jeweilige Mittelwert aus (PB1, PB2) bzw. (PD1, PD2) bezeichnet wird;

c) Mindestens einmaliges zeitversetztes Wiederholen der Schritte a) und b), wobei an denselben mindestens drei Drucksensoren wie in der vorangegangenen Messung gemessen wird und aus den gemessenen Drucksignalen dieselben Quotienten und/oder Differenzen berechnet werden;

d) Erstellen mindestens eines zeitlichen Trends aus den gemessenen Drucksignalen ausgewählt aus der Gruppe bestehend aus (PB1, PB2, PD1, PD2) und/oder den berechneten Differenzen und/oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM);

e) Bewerten des mindestens einen zeitlichen Trends, ob eine Änderung des mindestens einen zeitlichen Trends über einen Toleranzbereich hinaus eingetreten ist;

f) Erstellen eines Musters für die Bewertung des mindestens einen zeitlichen Trends;

g) Zuordnung des Musters zu einem Störungsbild und

h) Anzeigen der Störung auf einer Anzeigevorrichtung der zentralen Recheneinheit.

In einer besonders bevorzugten Ausführungsform umfasst das erfindungsgemäße Verfahren die Messung von vier Drucksignalen und stellt sich damit wie folgt dar. Verfahren zur Unterscheidung von Störungen und/oder Änderungen des Flusswiderstandes in einem Blutbehandlungssystem, das die folgenden Schritte umfasst:

a) Messung von vier Drucksignalen (PB1, PB2, PD1, PD2), die an vier Drucksensoren gemessen werden ([PB1], [PB2], [PD1] und [PD2]), wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;

b) Berechnung der Quotienten und/oder der Differenzen aus den gemäß Schritt a) gemessen Drucksignalen, wobei die Quotienten und/oder Differenzen berechnet werden aus den Drucksignalen (PB1, PB2) und (PB1, PD1) und (PB1, PD2) und (PB2, PD1) und (PB2, PD2) und (PD1, PD2) und (PB1, PDM) und (PB2, PDM) und (PD1, PBM) und (PD2, PBM) und (PDM, PBM) in einer zentralen Recheneinheit, wobei mit PBM oder PDM der jeweilige Mittelwert aus (PB1, PB2) bzw. (PD1, PD2) bezeichnet wird;

c) Mindestens einmaliges zeitversetztes Wiederholen der Schritte a) und b), wobei an denselben vier Drucksensoren wie in der vorangegangenen Messung gemessen wird und aus den gemessenen Drucksignalen dieselben Quotienten und/oder Differenzen berechnet werden;

d) Erstellen der zeitlichen Trends aus den gemessenen Drucksignalen (PB1, PB2, PD1, PD2) und/oder den berechneten Differenzen und/oder Quotienten der Drucksignale (PB1, PB2) und (PB1, PD1) und (PB1, PD2) und (PB2, PD1) und (PB2, PD2) und (PD1, PD2) und (PB1, PDM) und (PB2, PDM) und (PD1, PBM) und (PD2, PBM) und (PDM, PBM);

e) Bewerten der zeitlichen Trends, ob eine Änderung der zeitlichen Trends über einen Toleranzbereich hinaus eingetreten ist;

f) Erstellen eines Musters für die Bewertung der zeitlichen Trends;

g) Zuordnung des Musters zu einem Störungsbild und

h) Anzeigen der Störung auf einer Anzeigevorrichtung der zentralen Recheneinheit.

In dieser Ausführungsform werden in dem Blutbehandlungssystem vier Drucksignale PB1, PB2, PD1 und PD2 gemessen. Die Messung erfolgt an den Drucksensoren [PB1], [PB2], [PD1] und [PD2], wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck.

Aus den gemessenen Drucksignalen wird im nächsten Schritt in einer zentralen Recheneinheit jede mögliche Kombination aus Quotienten und/oder Differenzen berechnet, die sich aus den vier Drucksignalen bilden lässt. Das heißt konkret, dass Quotienten und/oder Differenzen aus den Drucksignalen (PB1, PB2), (PB1, PD1), (PB1, PD2), (PB2, PD1), (PB2, PD2), (PD1, PD2), (PB1, PDM), (PB2, PDM), (PD1, PBM), (PD2, PBM) und (PDM, PBM) berechnet werden. Somit liegen nun alle Quotienten und/oder Differenzen aus den Drucksignalen für die erste Messung vor.

Im nächsten Schritt wird eine erneute Messung an den vier Drucksensoren durchgeführt, welche zeitversetzt zu der ersten Messung stattfindet. Diese Messung erfolgt identisch zu der ersten Messung, d.h. an denselben Drucksensoren, mit den gleichen Einstellungen, nur mit dem Unterschied, dass die Drucksignale zeitversetzt zur ersten Messung gemessen werden. Aus den nun zeitversetzt gemessenen Drucksignalen wird analog zur oben beschriebenen Berechnung in einer zentralen Recheneinheit jede mögliche Kombination aus Quotienten und/oder Differenzen berechnet, die sich aus den vier zeitversetzt gemessenen Drucksignalen bilden lässt. Das heißt konkret, dass Quotienten und/oder Differenzen aus den zeitversetzt gemessenen Drucksignalen (PB1, PB2), (PB1, PD1) (PB1, PD2), (PB2, PD1), (PB2, PD2), (PD1, PD2), (PB1, PDM), (PB2, PDM), (PD1, PBM), (PD2, PBM) und (PDM, PBM) berechnet werden. Somit liegen nun die Quotienten und/oder Differenzen aus den zeitversetzt gemessenen Drucksignalen für die zweite Messung vor.

Allgemein sei angemerkt, daß die erfindungsgemäßen Verfahren bei der zuvor angeführten Kombination von Drucksignalen voraussetzen, daß bei wiederholten bzw. analogen Messungen und Berechnungen die gleiche Messung und Berechnung durchgeführt wird. Insbesondere soll die Bildung der Quotienten nicht einmal a/b und bei der erneuten Messung b/a umfassen, sondern kontinuierlich a/b. Das gleiche gilt *mutatis mutandis* für die Bildung der Differenzen: wenn bei der ersten Messung a - b gebildet wird, wird bei der erneuten Messung ebenfalls a - b und nicht b - a gebildet.

In weiteren zeitversetzten Messungen können nun weitere Drucksignale gemessen werden und weitere Quotienten und/oder Differenzen aus den Drucksignalen berechnet werden, erfindungsgemäß reichen aber die oben genannten zwei Messungen aus, um das Verfahren auszuführen. Zur Ermittlung eines Trends können theoretisch beliebig viele Messungen zu unterschiedlichern Zeiten einbezogen werden, wobei der Zeitraum, welcher für die Ermittlung eines Trends ausgewählt wird, deutlich unter einem Zehntel (1/10) der Dauer der Dialysesitzung liegen sollte, vorzugsweise unter einem Fünfzigstel (1/50), weiter bevorzugt unter 1/80, weiter bevorzugt unter 1/100, weiter bevorzugt unter 1/110, weiter bevorzugt unter 1/120 Dauer der Dialysesitzung liegen sollte. Innerhalb des betrachteten Zeitraums für die Ermittlung eines Trends können 2 bis 60.000 zeitlich versetzte Messungen von Drucksignalen liegen, vorzugsweise 3 bis 20.000, weiter bevorzugt 4 bis 10.000, noch weiter bevorzugt 5 bis 5.000 Messungen.

Aus den gemessenen Drucksignalen der ersten und zweiten Messung und allen Quotienten und/oder Differenzen der ersten Messung und zweiten Messung wird nun ein zeitlicher Trend berechnet, d.h. dass der Quotient und/oder die Differenz aus den Drucksignalen (PB1, PB2) aus der ersten Messung ins Verhältnis gesetzt wird mit dem Quotienten und/oder der Differenz aus den Drucksignalen (PB1, PB2) aus der zweiten Messung. Dieser zeitliche Trend wird für jeden der Quotienten und/oder Differenzen aus den Drucksignalen der ersten Messung und den Quotienten und/oder Differenzen aus den Drucksignalen der zweiten Messung erstellt. Weiterhin wird der zeitliche Trend für jedes der Drucksignale PB1, PB2, PD1 und PD2 erstellt. Somit liegen nun zeitliche Trends für die gemessenen Drucksignale und jeden Quotienten und/oder Differenzen der Drucksignale aus der ersten und der zweiten Messung vor. Jeder zeitliche Trend wird dahingehend bewertet, ob eine Änderung über einen Toleranzbereich hinaus eingetreten ist. Dabei kann jeder Trend dahingehend eingeordnet werden, ob keine Veränderung eingetreten ist, die über einen Toleranzbereich hinausgeht, ob der Trend aufsteigend ist, d.h. den Toleranzbereich an der oberen Grenze durchstößt oder ob der Trend absteigend ist, d.h. den Toleranzbereich an der unteren Grenze durchstößt. Aus der Bewertung jedes zeitlichen Trends ergibt sich damit ein Muster, welches einem spezifischen Störungsbild zugeordnet werden kann.

Beispielsweise könnte die Messung der Drucksignale zum Zeitpunkt t=0s und zu weiteren zeitversetzen Messungen an den Zeitpunkten t=60s, t=120s, t=180s, t=240s und t=300s die Folgenden Werte ergeben haben:

|  | PB1 [mmHG] | PB2 [mmHG] | PD1 [mmHg] | PD2 [mmHG] |
|---|---|---|---|---|
| t =0s | 120 | 50 | 80 | 70 |
| t =60s | 125 | 49 | 79 | 70 |
| t =120s | 133 | 51 | 78 | 70 |
| t =180s | 139 | 48 | 80 | 69 |
| t =240s | 145 | 52 | 81 | 70 |
| t =300s | 150 | 50 | 80 | 71 |

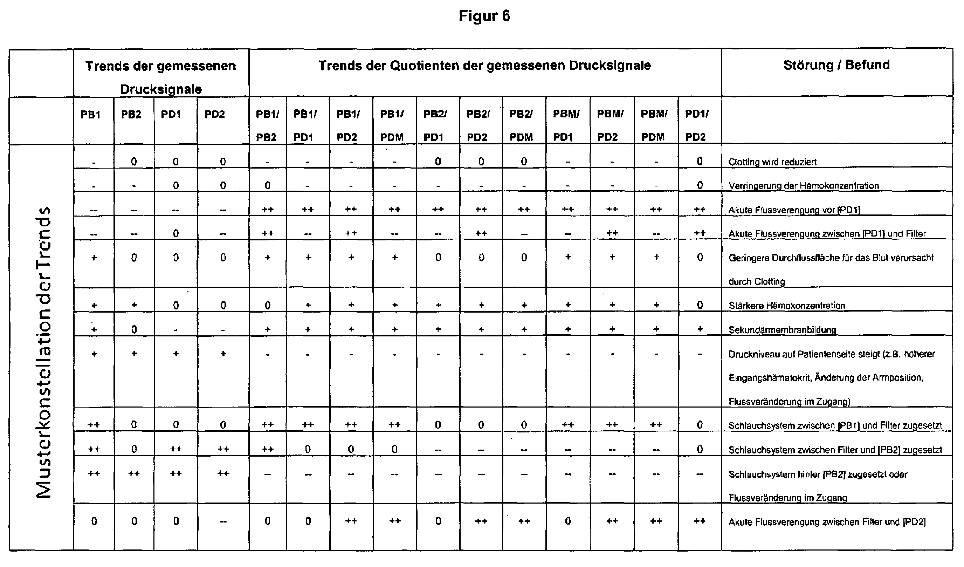

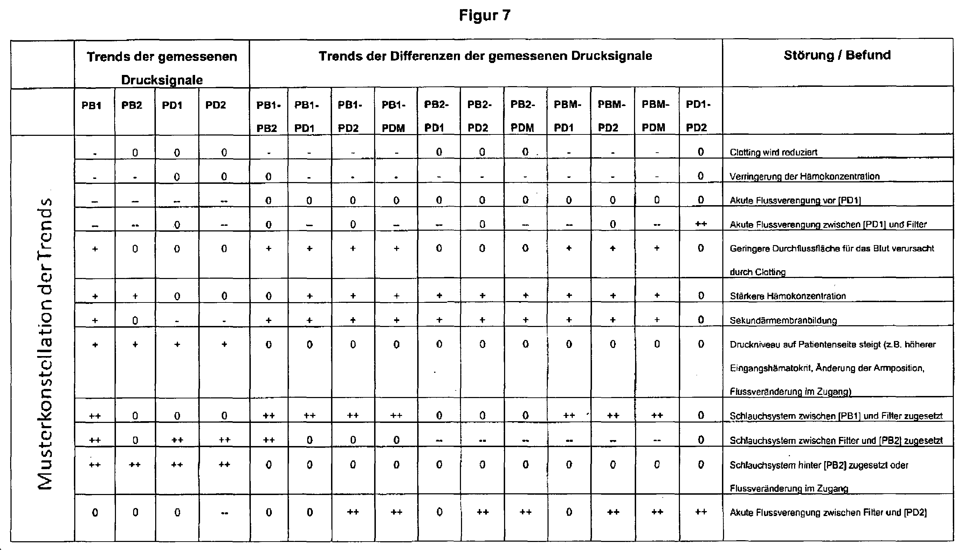

Aus den gemessenen Werten werden nun für jede der zeitversetzten Messungen alle möglichen Quotienten und/oder Differenzen gebildet. Für den Zeitpunkt t=0 würde dies bedeuten, dass Quotienten und/oder Differenzen aus (PB1, PB2), (PB1, PD1) (PB1, PD2), (PB2, PD1), (PB2, PD2), (PD1, PD2), (PB1, PDM), (PB2, PDM), (PD1, PBM), (PD2, PBM) und (PDM, PBM) berechnet werden. Dies würde für den Zeitpunkt t=0 zu folgendem Ergebnis für die Quotientenbildung führen: (120/50), (120/80), (120/70), (50/80), (50/70), (80/70), (120/75), (50/75), (80/85), (70/85) und (75/85). Die Berechnung erfolgt auch für die Differenzen und wird für jede der zeitversetzten Messungen durchgeführt. Aus den nun berechneten Quotienten und/oder Differenzen und/oder den absolut gemessenen Werten werden nun Trends für die absoluten Werte und/oder die Quotienten und/oder Differenzen erstellt. Für den Quotienten aus (PB1, PB2) könnte sich hiermit der Folgende zeitliche Trend ergeben: (120/50), (125/49), (133/51), (139/48), (145/52) und (150/50). Dieser zeitliche Trend wird für jeden Quotienten und/oder der Differenzen und/oder der absoluten Werte erstellt. Hieraus ergibt sich im vorliegenden Beispiel, dass der zeitliche Trend t=0 bis t=300s für die Quotienten aus (PB1, PB2), (PB1, PD1) (PB1, PD2), (PB1, PDM), (PBM, PD1), (PBM, PD2) und (PBM/PDM) ansteigend ist, wobei die übrigen zeitlichen Trends der Quotienten unverändert bleiben. Weiterhin ist es möglich, Trends innerhalb von Trends zu erstellen. Dies würde im vorliegenden Beispiel bedeuten, dass nicht nur ein zeitlicher Trend zwischen t=0 bis t=300s erstellt wird, sondern, dass auch eine beliebige Anzahl an zeitlichen Trends zwischen t=0 und t=240s, oder t=60s und t=180s usw. erstellt werden können. Die Erstellung von längerfristigen Trends eignet sich naturgemäß besser zur Erfassung von Störungen, welche eher schleichend auftreten, wobei mit kürzeren Trends auch Störungen erkannt werden können, die kleinerer Natur sind. Die in diesem Beispiel gezeigte Musterkonstellation aus den Trends der Quotienten und/oder der Differenzen und/oder der absoluten Werte ist spezifisch für eine Sekundärmembranbildung. Für alle möglichen Störungsbilder gibt es nun spezifische Musterkonstellationen, die sich aus der Bildung der Trends aus den Quotienten und/oder der Trends der Differenzen und/oder der Trends der absolute Werte bilden lassen. Beispielhaft sind solche Musterkonstellationen in den Figuren 6 und 7 aufgeführt.

In einer weiteren Ausführungsform erfolgt die Messung der mindestens zwei Drucksignale zeitgleich. In dieser Ausführungsform wird Schritt a) durch den folgenden Schritt a') ersetzt:

a') Messung von mindestens zwei Drucksignalen ausgewählt aus der Gruppe bestehend aus (PB1, PB2, PD1, PD2), die zeitgleich an mindestens zwei Drucksensoren gemessen werden, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;

Mit "zeitgleich", wie hierin verwendet, ist gemeint, dass die Drucksignale gleichzeitig bzw. unmittelbar hintereinander gemessen werden. D.h., dass keine längere Pause zwischen der Messung des einen Drucksignals und des oder der anderen Drucksignale liegt. Bevorzugterweise erfolgt die Messung der Drucksignale innerhalb einer Periode von 5 Minuten, bevorzugt von 1 Minute, noch bevorzugter innerhalb von 45 Sekunden, noch bevorzugter innerhalb von 30s, weiterhin bevorzugt innerhalb von 15s und am meisten bevorzugt innerhalb von Perioden kleiner oder gleich 1 s.

Prinzipiell kann das erfindungsgemäße Verfahren mit mindestens zwei Drucksensoren ausgeführt werden, wobei bevorzugterweise drei Drucksensoren und weiterhin bevorzugt vier Drucksensoren verwendet werden.

Der Tangentialflussfilter ist einer der gängigsten Filtertypen, der in einer Vorrichtung zur Behandlung von Blut eingesetzt werden kann. Prinzipiell können auch andere Filtertypen zum Einsatz kommen, ohne dass das erfindungsgemäße Verfahren in irgendeiner Weise beeinträchtigt wäre. Daher bezieht sich die Erfindung nicht nur auf Dialysegeräte mit einem Tangentialflussfilter, sondern mit jedwedem Dialysator-Typ.

Erfindungsgemäß werden die Drucksignale gemessen und alle möglichen Quotienten und/oder Differenzen aus den gemessenen Drucksignalen berechnet, d.h. beim Einsatz von zwei Drucksensoren werden zwei Drucksignale gemessen, aus denen der Quotient und/oder die Differenz gebildet werden. Das heißt, daß bei der Verwendung der Drucksensoren [PB1] und [PD1] die Drucksignale PB1 und PD2 gemessen und die Quotienten und/oder die Differenzen aus (PB1, PD1) errechnet werden. Werden drei Drucksensoren eingesetzt, dann wird an jedem dieser Drucksensoren ein Drucksignal gemessen, für welches dann mit jedem der anderen Drucksignale ein Quotient bzw. die Differenz berechnet werden. Zum Beispiel könnten die Drucksignale PB1, PB2 und PD1 gemessen werden, aus denen dann die Quotienten und/oder Differenzen aus (PB1, PB2), (PB1, PD1), (PB2, PD1) und (PBM, PD1) berechnet werden. Für den Fall, dass vier Drucksensoren eingesetzt werden, wird der Quotient und/oder die Differenz für jedes mögliche Paar der vier Drucksignale berechnet. Zusätzlich werden Mittelwerte für die beiden dialysatseitigen Drucksignale und die beiden blutseitigen Drucksignale berechnet, für welche dann wieder mit jedem der Drucksignale Quotienten und/oder Differenzen berechnet werden, d.h. konkret, dass die Quotienten und/oder Differenzen aus (PB1, PB2), (PB1, PD1) (PB1, PD2), (PB2, PD1), (PB2, PD2), (PD1, PD2), (PB1, PDM), (PB2, PDM), (PD1, PBM), (PD2, PBM) und (PDM, PBM) berechnet werden. Mit PBM oder PDM wird der jeweilige Mittelwert aus blutseitigen und dialysatseitigen Drucksignalen bezeichnet.

Die mindestens einmalige zeitversetzte Messung erfolgt jeweils mit derselben Anzahl und denselben Drucksensoren, wie bei der vorangegangenen Messung eingesetzt wurden. Die zeitversetzte Messung erfolgt dabei unter den gleichen Bedingungen, d.h. mit denselben Einstellungen, mit dem einzigen Unterschied, dass sie zeitlich versetzt erfolgt. Somit erhält man durch die zeitlich versetzte Messung für jeden der Drucksensoren ein weiteres zeitversetztes Drucksignal. Erfindungsgemäß muss mindestens eine zeitversetzte Messung erfolgen. In der Praxis hat sich erwiesen, dass mehrere zeitversetzte Messungen vorteilhaft sind, um Störungen des Flusswiderstandes in einem Blutbehandlungssystem zu detektieren. Diese erfolgen vorteilhafterweise über die gesamte Dauer der Behandlung mit dem Blutbehandlungssystem, um sicherzustellen, dass auch über die gesamte Dauer Störungen im System erkannt werden können. Somit bedeutet zeitversetzt, dass zwischen jeder Messung ein gewisser Zeitraum liegt, der individuell festgelegt werden kann. Hierbei kann es sich um sehr schnelle Messungen handeln, wie z.B. 20 mal pro Sekunde, aber es ist auch möglich, dass die Messungen über einen größeren Zeitraum durchgeführt werden, wie z.B. alle 5 Minuten eine Messung, jede Minute eine Messung, alle 30s eine Messung, alle 10s eine Messung, jede Sekunde eine Messung oder jede Zehntel Sekunde eine Messung.

Bevorzugterweise werden die gemessenen Drucksignale normalisiert, um die normalen Druckschwankungen, welche z.B. durch die Pumpe verursacht werden, zu eliminieren.

Die Erstellung des zeitlichen Trends beinhaltet den Vergleich der gemessenen Drucksignale und/oder der Quotienten und/oder der Differenzen aus den Messungen, dahingehend, ob sich Änderungen in den gemessenen Drucksignalen und/oder im Quotienten und/oder den Differenzen über die Zeit ergeben haben. Für die Erstellung des zeitlichen Trends müssen erfindungsgemäß mindestens zwei zeitversetzte Messungen an den Drucksensoren erfolgen. Die zeitlichen Trends werden dabei für jeden der gemessenen Drucksignale und/oder der Quotienten und/oder der Differenzen erstellt, d.h. bei vier Drucksensoren und damit vier gemessenen Drucksignalen werden aus den vier gemessenen Drucksignalen alle möglichen Quotienten und/oder Differenzen gebildet. Erfindungsgemäß werden mindestens einmal zeitversetzt an den vier Drucksensoren die vier Drucksignale gemessen, aus denen erneut Quotienten und/oder Differenzen gebildet werden. Jeder der gemessenen Drucksignale und/oder der Quotienten und/oder der Differenzen aus der ersten Messung wird nun mit den gemessenen Drucksignalen und/oder den Quotienten und/oder den Differenzen aus der zeitversetzten Messung verglichen, somit ergibt sich für jeden der gemessenen Drucksignale und/oder den Quotienten und/oder den Differenzen ein zeitlicher Trend.

Sind mehrere zeitversetzte Messungen erfolgt, liegen erfindungsgemäß für jeden dieser Zeitpunkte mehrere gemessene Drucksignale und/oder Quotienten und/oder Differenzen vor. Es liegt im Ermessen des Fachmanns, ob bevorzugterweise "kurze" zeitliche Trends erfasst werden sollen, die nur aus wenigen zeitlich versetzten Messungen bestehen, oder ob längerfristige zeitliche Trends erfasst werden sollen, die aus einer Vielzahl von zeitversetzen Messungen bestehen. Die Erstellung des zeitlichen Trends kann prinzipiell auch durch den Vergleich der gemessenen Drucksignale und/oder der Quotienten und/oder Differenzen über alle zeitversetzten Messungen erfolgen. Es ist aber auch möglich, dass der zeitliche Trend nur für einige dieser zeitversetzen Messungen erstellt wird. Konkret bedeutet dies, dass kleine Abweichungen prinzipiell besser durch die Erstellung von "kurzen" Trends erkannt werden können, welche nur aus wenigen zeitversetzten Messungen bestehen, wobei schleichende, kontinuierliche Veränderungen im System besser durch die Erstellung von längerfristigen Trends erkannt werden können, welche eine Vielzahl von zeitversetzt gemessenen Drucksignalen und/oder Quotienten und/oder Differenzen enthalten. Erfindungsgemäß können sowohl kurze als auch längerfristige zeitliche Trends erstellt und miteinander kombiniert werden.

In der Praxis hat sich gezeigt, dass mit der Erstellung der zeitlichen Trends erst nach einer Vorlaufzeit von ca. 1 - 5 Minuten begonnen werden sollte, da abhängig vom Hämatokrit, der Ultrafiltrationsrate, des spezifischen Filters, der benutzt wird, d.h. insbesondere der Anzahl der Fasern im Filter, der Flussgeschwindigkeit und generell der Viskosität des Blutes, es eine Weile dauert, bis stabile Drucksignale gemessen werden können, aus denen dann die zeitlichen Trends für die gemessenen Drucksignale und/oder Quotienten und/oder Differenzen erstellt werden können.

Die Bewertung der zeitlichen Trends erfolgt dadurch, dass festgestellt wird, ob eine Änderung des zeitlichen Trends über einen Toleranzbereich hinaus eingetreten ist. Hierbei wird unterschieden zwischen zeitlichen Trends, welche schnell ansteigen (++), ansteigen (+), unverändert bleiben (0), abfallen (-) und solche die schnell abfallen (--).

Der Toleranzbereich kann z.B. so eingegrenzt sein, dass bei einem kleinen zeitlichen Trend, bestehend aus den gemessenen Drucksignalen und/oder den Quotienten und/oder den Differenzen zweier zeitversetzter Messungen, eine Änderung der gemessenen Drucksignale und/oder der Quotienten und/oder Differenzen um 10%, in die eine oder andere Richtung, entweder als ein Ansteigen (+) oder ein Abfallen (-) des zeitlichen Trends bewertet werden. Dahingegen werden jegliche Schwankungen innerhalb dieses Toleranzbereiches als "unverändert geblieben (0)" bewertet. Somit gibt der Toleranzbereich den Bereich an, in dem die Werte der gemessenen Drucksignale und/oder der Quotienten und/oder der Differenzen liegen können, um als "unverändert geblieben (0)" bewertet zu werden. Werte, die außerhalb des Toleranzbereiches liegen, sind damit verändert, womit der zeitliche Trend dann entsprechend bewertet wird. Damit ein zeitlicher Trend als schnell ansteigend (++) bzw. schnell abfallend (--) bewertet wird, muss es zu einer akuten Änderung zwischen den zeitversetzt ermittelten gemessenen Drucksignalen und/oder der Quotienten und/oder Differenzen kommen, d.h., die Änderung darf sich nicht über einen längeren Zeitraum entwickeln, sondern muss schlagartig auftreten. Dies ist insbesondere dann der Fall, wenn z.B. ein Schlauch abgeknickt ist. Die Schwelle zur Abgrenzung zwischen ansteigenden bzw. abfallenden sowie schnell ansteigenden bzw. schnell absteigenden Signalen kann nach Gerätetyp, Patient, bevorzugten Auswertungsparametern etc. individuell festgelegt werden.

Die Begrenzung des Toleranzbereiches ist von einer gewissen Anzahl von Faktoren abhängig, wie z.B. vom Hämatokrit, der Ultrafiltrationsrate, des spezifischen Filters der benutzt wird, d.h. insbesondere der Anzahl der Fasern im Filter, der Flussgeschwindigkeit und generell der Viskosität des Blutes. Der Toleranzbereich kann z.B. derart eingegrenzt sein, dass die gemessenen Drucksignale und/oder Quotienten und/oder Differenzen innerhalb des zeitlichen Trends einen absoluten Wert nicht übersteigen oder unterschreiten dürfen. Andererseits kann der Toleranzbereich auch so gewählt werden, dass z.B. eine gewisse prozentuale Änderung der gemessenen Drucksignale und/oder Quotienten und/oder Differenzen nicht überschreiten darf. Naturgemäß können mehrere Grenzen für Toleranzbereiche miteinander kombiniert werden, indem z.B. festgelegt wird, dass der Toleranzbereich sowohl durch einen oberen absoluten Wert begrenzt wird als auch durch prozentuale Änderungen der gemessenen Drucksignale und/oder der Quotienten und/oder der Differenzen. In solch einem Fall könnte somit ein zeitlicher Trend z.B. als ansteigend (+) bewertet werden, in dem er entweder die obere absolute Grenze oder die Grenze der prozentualen Änderungen der gemessenen Drucksignale und/oder der Quotienten und/oder der Differenzen übersteigt. Es liegt im Können des Fachmanns, Toleranzbereiche festzulegen, ohne erfinderisch tätig werden zu müssen.

Aus der Bewertung der zeitlichen Trends ergibt sich ein Muster, welches einem spezifischen Störungsbild zugeordnet werden kann. Somit kann sich z.B. ergeben, dass der Quotient aus PB1/PD1 einen abfallenden Trend hat, wobei der Quotient aus PB2/PD2 einen gleichbleibenden Trend aufweist. Die Gesamtheit der bewerteten zeitlichen Trends ergibt dabei ein Muster. In den Fig. 6 - 7 sind beispielhaft einige Musterkonstellationen für auftretende Druckveränderungen im Betrieb einer Dialysemaschine und die ihnen zugrundeliegenden Störungen zusammengestellt.

Besonders bevorzugt ist es, wenn mindestens ein Drucksignal auf der Blutseite und mindestens ein weiteres Drucksignal auf der Dialysatseite ermittelt werden. Weiter bevorzugt ist es, wenn ein Drucksignal auf der Blutseite und zwei Drucksignale auf der Dialysatseite oder zwei Drucksignale auf der Blutseite und ein Drucksignal auf der Dialysatseite ermittelt werden. In einer insbesondere bevorzugten Ausführungsform werden zwei Drucksignale auf der Blutseite und zwei Drucksignale auf der Dialysatseite ermittelt. Die Druckmessungen erfolgen dabei vorzugsweise an oder unmittelbar vor den Ein- und Auslässen des Tangentialflussfilters (TFF).

Bei den gemessenen Drucksignalen kann es sich um Absolutdrücke, relative Drücke, absolute Druckdifferenzen zwischen zwei Druckmesspunkten, relative Druckdifferenzen zwischen zwei Druckmesspunkten, absoluten Druckamplituden, relativen Druckamplituden, Differenzen zwischen den absoluten Druckamplituden an zwei Druckmesspunkten oder Differenzen zwischen den relativen Druckamplituden an zwei Druckmesspunkten oder eine Kombination davon oder die Frequenzspektra der Drücke handeln.

Der Begriff "absolutdruck" oder "Absolutdrücke", wie hierin verwendet, beschreibt den Druck gegenüber dem Atmosphärendruck.

Der Begriff "relativer Druck" oder "relative Drücke", wie hierin verwendet, beschreibt die relative Änderung eines Drucksignals in Bezug auf ein zweites Drucksignal.

Der Begriff "Druckdifferenz" oder "Druckdifferenzen", wie hierin verwendet, beschreibt die Differenz zweier Drücke.

Der Begriff "Druckamplitude" oder "Druckamplituden", wie hierin verwendet, beschreibt den ermittelten oder gemessenen Wert der Druckschwankungen. Synonym dazu kann der Begriff Druckamplitudenhub verwendet werden.

Der Begriff "Frequenzspektrum" oder "Frequenzspektra", wie hierin verwendet, bezeichnet die Gesamtheit der Frequenzen, die von einem schwingenden System erzeugt werden bzw. in einem Signal enthalten sind.

Dabei hat sich überraschend herausgestellt, dass die Messung und Auswertung der Drucksignale, z.B. erzeugt auch durch eine Blutpumpe P im System, Aufschluss über die Flusseigenschaften in einer Vorrichtung zur Behandlung von Blut gibt, indem die Quotienten und/oder Differenzen aus den gemessenen Drucksignalen gebildet werden, aus denen ein zeitlicher Trend abgeleitet wird, der ein spezifisches Muster für spezielle Störungsbilder aufweist. Erfindungsgemäß kann jedes Drucksignal im System unabhängig seines Ursprungs zur Auswertung benutzt werden. Beispielsweise werden in einer Ausführungsform die Drucksignale, die durch das Schalten einer Bilanzkammer BK entstehen, ermittelt und für die Auswertung genutzt.

Die Überwachung der Systemeigenschaften während der Behandlung wird vorzugsweise derart umgesetzt, dass die durch die Blutpumpe P erzeugten Drucksignale und deren Propagation überwacht werden. Erfindungsgemäß können auch die Drucksignale einer dialysatseitigen Pumpe oder die durch das Schalten von Ventilen erzeugten Druckspitzen überwacht werden. Es ist auch möglich, dass kombinierte Drucksignal aus beiden Pumpen zu überwachen, oder jedes Drucksignal einzeln zu erfassen. Insbesondere bevorzugt ist die Überwachung von den 4 Drucksignalen an den Eingängen und Ausgängen des Tangentialflussfilters TFF.

Je nach den Systemeigenschaften des Blutbehandlungssystems breiten sich die Drucksignale in dem System entlang der Blutflussrichtung und in Transmembranrichtung aus.

Es ist also möglich, durch die Überwachung der Drucksignale auf der Blutseite und der Dialysatseite sowie durch die Überwachung der Verhältnisse dieser Drücke, Änderungen der Flussbedingungen zu registrieren und zwischen Veränderungen in der Blutflussrichtung, Dialysatflussrichtung und Transmembranrichtung zu differenzieren.

Der Begriff "*Blutbehandlungseinheit*" beschreibt dabei eine Vorrichtung, die zur Reinigung und/oder Behandlung von Blut eingesetzt werden kann, deren Kernstück ein Tangentialflussfilter ist. Insbesondere kann es sich um eine Dialyseeinheit handeln, die zur Hämodialyse, Hämoperfusion, Hämofiltration oder Hämodiafiltration in der Lage ist.

Der Begriff "*Systemänderung*", wie hierin verwendet, umfasst unter anderem die Interaktion von Bestandteilen der Vorrichtung zur Behandlung von Blut, insbesondere der Filtermembran mit Blut. Durch diese Wechselwirkung verschlechtern sich die Flusseigenschaften sowohl in Transmembranrichtung als auch in Blutflussrichtung. Dies wird beispielsweise durch Thrombozytenanlagerung, Koagelbildung, chemisches Binden von Blutbestandteilen an die Membran oder schlicht mechanisches (strömungsbedingtes) Drücken der Blutbestandteile an und sogar in die Membran verursacht, ist aber nicht auf diese beschränkt und kann auch an anderen Stellen innerhalb der Blutbehandlungseinheit auftreten. Weiterhin umfasst sind Änderungen im System, wie sie z.B. durch das Abknicken eines Schlauches oder einem Leck im System auftreten können. Auch hierbei handelt es sich um Systemänderungen, obwohl keine direkte Interaktion von Bestandteilen der Vorrichtung mit Blut erfolgt.

Die Ermittlung der Drucksignale erfolgt über Drucksensoren. Hierfür können die aus dem Stand der Technik bekannten Drucksensoren benutzt werden, wie z.B. piezoresistive, piezoelektrische, frequenzanaloge, Drucksensoren mit Hallelementen, kapazitive, induktive und/oder Kombinationen davon. Bevorzugt sind hierbei Drucksensoren, deren Abtastrate mindestens 20 Hz beträgt. Die Abtastrate bezeichnet hierbei die Rate mit der Signalwerte aus einem kontinuierlichen Signal entnommen werden.

Die erfindungsgemäßen Zusammenhänge präsentieren sich im Detail wie in den Figuren 6 und 7 dargestellt:

Hat sich z.B. die Filtermembran durch Clotting zugesetzt, zeigt sich am Drucksensor [PB1] ein ansteigender Trend des gemessenen Drucksignals PB1, wobei die Trends der anderen Drucksignale PB2, PD1 und PD2 unverändert bleiben. Durch die weitere Berechnung der Quotienten und/oder der Differenzen der gemessenen Drucksignale, und der Erstellung der zeitlichen Trends ergibt sich ein weitaus differenzierteres Bild, welches zeigt, dass die zeitlichen Trends der Quotienten und/oder Differenzen aus (PB1, PB2), (PB1, PD1), (PB1, PD2), (PBM, PD1), (PDM, PD2), (PBM/PDM) ansteigen, wohingegen die zeitlichen Trends der Quotienten und/oder Differenzen aus (PB2, PD1), (PB2, PD2), (PB2, PDM) und (PD1, PD2) unverändert bleiben. Aus der Ganzheit der Trends der gemessenen Drucksignale und/oder der Trends der Quotienten und/oder der Trends der Differenzen der gemessenen Drucksignale ergibt sich eine charakteristische Musterkonstellation, welche direkt dem Störungsbild Clotting zugeordnet werden kann.

Der einzelne Wert (Drucksignal) an sich ist vielen Einflüssen unterworfen und man erhält durch die Betrachtung des Momentanwertes wenige Informationen. Daher wurde erfingdungsgemäß erkannt, dass erst durch die Bildung der Quotienten und/oder Differenzen der Drucksignale und der Erstellung eines zeitlichen Trends Störungen im Detail erfasst werden kann. Veränderungen in diesem zeitlichen Trend sind bei verschiedenen Störungsbildern unterschiedlich und ergeben ein spezifisches Muster, welches dem jeweiligen Störungsbild zugeordnet werden kann. Es lassen sich z.B. Systemänderungen bedingt durch die Behandlung von solchen durch äußere Einflüsse unterscheiden.

Die Differenzierung zwischen der Bildung von Quotienten und Differenzen der Drucksignale macht daher Sinn, da bei einer Druckänderung durch einen Offset, wie beispielsweise einem erhöhten Rückflusswiderstand sich die Drucksignale um den gleichen Absolutwert ändern, was dazu führt, dass zwar die Differenzen konstant bleiben, aber die Quotienten sich dennoch ändern, wodurch auch solche Störungen erfindungsgemäß erkannt werden können.

Weitere Störungen könnten z.B. in Form einer akuten Flussverengung vor [PD1] oder einer akuten Flussverengung zwischen [PD1] und Filter vorkommen. Erfindungsgemäß kann hier zwischen den beiden Störungen unterschieden werden. Bei einer akuten Flussverengung vor [PD1] zeigt sich an allen Drucksensoren ein schnell abfallender Trend der gemessenen Drucksignale. Erst durch die weitere Berechnung der Quotienten und der Differenzen der gemessenen Drucksignale und der Erstellung der zeitlichen Trends ergibt sich ein weitaus differenzierteres Bild, welches zeigt, dass die zeitlichen Trends der Quotienten der gemessenen Drucksignale alle einen ansteigenden Trend aufweisen, wohingegen die zeitlichen Trends der Differenzen keine Veränderung zeigen. Aus der Ganzheit der Trends der gemessenen Drucksignale und der Trends der Quotienten und der Trends der Differenzen der gemessenen Drucksignale ergibt sich jetzt die charakteristische Musterkonstellation, welche direkt dem Störungsbild akute Flussverengung vor [PD1] zugeordnet werden kann.

Die Störung akute Flussverengung zwischen [PD1] und Filter hingegen zeichnet sich dadurch aus, dass bei einer akuten Flussverengung zwischen [PD1] und Filter an allen Drucksensoren ein schnell abfallender Trend der gemessenen Drucksignale gezeigt wird, außer am Drucksensor [PD1], welcher unverändert bleibt. Die weitere Berechnung der Quotienten und der Differenzen der gemessenen Drucksignale und die Erstellung der zeitlichen Trends zeigt in diesem Fall, dass die zeitlichen Trends der Quotienten der gemessenen Drucksignale (PB1, PB2), (PB1, PD2), (PB2, PD2), (PBM, PD2) und (PD1, PD2) ansteigen, wohingegen die zeitlichen Trends der Quotienten der gemessenen Drucksignale (PB1, PD1), (PB1, PDM), (PB2, PD1), (PB2, PDM), (PBM, PD1) und (PBM, PDM) abfallen. In diesem Zusammenhang zeigen die zeitlichen Trends der Differenzen ein anderes Bild. Hier steigt die Differenz der zeitlichen Trends (PD1, PD2) schnell an, die Differenzen der zeitlichen Trends aus (PB1, PB2), (PB1, PD2), (PB2, PD2) und (PBM, PD2) bleiben unverändert, wohingegen die Differenzen der zeitlichen Trends aus (PB1, PD1), (PB1, PDM), (PB2, PD1), (PB2, PDM), (PBM, PD1) und (PBM, PDM) schnell abfallen. Aus der Ganzheit der Trends der gemessenen Drucksignale und der Trends der Quotienten und der Trends der Differenzen der gemessenen Drucksignale ergibt sich jetzt die charakteristische Musterkonstellation, welche direkt dem Störungsbild akute Flussverengung zwischen [PD1] und Filter zugeordnet werden kann.

Erfindungsgemäß können nicht nur Störungen in dem Sinne erkannt und qualitativ bewertet werden, sondern es ist auch möglich, dass Befunde, welche keine Störung sind, zu detektieren und qualitativ zu bewerten. Bei diesen Befunden handelt es sich erfindungsgemäß auch um Systemänderungen, welche zwar keine Störungen darstellen, wobei es aber es dennoch sinnvoll sein kann, diese Befunde zu erfassen und dem Bediener des Blutbehandlungssystems zu melden.

Um solch einen Befund handelt es sich z.B. wenn durch eine Maßnahme das Clotting reduziert werden konnte. Die Reduzierung des Clottings stellt sich erfindungsgemäß so dar, dass an dem Drucksensor [PB1] ein abfallender Trend der gemessenen Drucksignale ermittelt wird, wohingegen die gemessenen Drucksignale der anderen Drucksensoren einen zeitlich unveränderten Trend aufweisen. Durch die weitere Berechnung der Quotienten und/oder der Differenzen der gemessenen Drucksignale und der Erstellung der zeitlichen Trends ergibt sich ein weitaus differenzierteres Bild, welches zeigt, dass die zeitlichen Trends der Quotienten und/oder Differenzen aus (PB1, PB2), (PB1, PD1), (PB1, PD2), (PBM, PD1), (PDM, PD2) und (PBM/PDM) abfallen, wohingegen die die zeitlichen Trends der Quotienten und/oder Differenzen aus (PB2, PD1), (PB2, PD2), (PB2, PDM) und (PD1, PD2) unverändert bleiben. Aus der Ganzheit der Trends der gemessenen Drucksignale und der Trends der Quotienten und/oder der Trends der Differenzen der gemessenen Drucksignale ergibt sich eine charakteristische Musterkonstellation, welche direkt dem Befund "Clotting reduziert" zugeordnet werden kann.

Solche Befunde stellen damit wertvolle Informationen für den Bediener des Blutbehandlungssystems dar, da sie unter anderem Aufschluss darüber geben können, ob eine eingeleitete Maßnahme auch zum Erfolg geführt hat.

Der Transmembrane Flusswiderstand ermittelt sich aus der Differenz der gemessenen Drucksignale aus PB1 und PD2 und/oder dem Verhältnis aus PB1 und PD2 und/oder der Differenz aus PB2 und PD2 und/oder dem Verhältnis aus PD2 und PB2. Weiterhin dienen Änderungen der Druckamplituden $A_{PD2}$ und/oder dem Verhältnis der Druckamplituden $A_{PB1}$ und $A_{PD2}$ und/oder dem Verhältnis der Druckamplituden $A_{PB2}$ und $A_{PD2}$ und/oder der Differenz aus PB1 und PD1 und/oder dem Verhältnis aus PB1 und PD1 und/oder der Differenz aus PB2 und PD1 und/oder dem Verhältnis aus PB2 und PD1 und/oder der Änderung der Druckamplitude $A_{PD1}$ und/oder dem Verhältnis der Druckamplituden $A_{PB1}$ und $A_{PD1}$ und/oder dem Verhältnis der Druckamplituden $A_{PB2}$ und $A_{PD1}$ als Indikator für den transmembranen Flusswiderstand.

Mit [PB1] wird der Drucksensor im Blutkreislauf vor dem Bluteinlass in den Tangentialflussfilters TFF bezeichnet und mit PB1 der am Drucksensor [PB1] gemessene Druck. Mit [PB2] wird der Drucksensor im Blutkreislauf nach dem Blutauslass aus dem Tangentialflussfilters TFF bezeichnet und mit PB2 der am Drucksensor [PB2] gemessene Druck. Mit [PD1] wird der Drucksensor im Dialysatkreislauf vor dem Dialysateinlass in den Tangentialflussfilters TFF bezeichnet und mit PD1 der am Drucksensor [PD1] gemessene Druck. Mit [PD2] wird der Drucksensor im Dialysatkreislauf nach dem Dialysatauslass aus dem Tangentialflussfilters TFF bezeichnet und mit PD2 der am Drucksensor [PD2] gemessene Druck.

Mit PBM oder PDM wird der jeweilige Mittelwert aus PB1 und PB2 bzw. PD1 und PD2 bezeichnet.

Bisherige Lösungsansätze konnten nicht zwischen Veränderungen in Blutflussrichtung, Dialysatflussrichtung und Transmembran-Richtung differenzieren, wodurch eine gezielte Behebung von Störungen nicht möglich war. Durch die Verwendung von bis zu vier Drucksensoren und der Auswertung der erfassten Drucksignale an jedem dieser Drucksensoren, können alle oben genannten Verhältnisse überwacht werden. Aus der Gesamtheit der Drucksignale kann eine vollständige Auswertung erfolgen, die es ermöglicht, die Art der Systemänderung gezielt zu bestimmen. Durch eine geeignete Auswertung von vier Drucksignalen läßt sich auch die Lage der Störung zumindest eingrenzen.

Bevorzugt werden die Drucksignale blutseitig am Drucksensor [PB1] sowie am Drucksensor [PB2] ermittelt. Dialysatseitig werden die Drucksignale bevorzugt am Drucksensor [PD2] und am Drucksensor [PD1] ermittelt.

In einer alternativen Ausführungsform ist es jedoch ausreichend, wenn die Drucksignale PB1 und PB2 und eines der dialysatseitigen Signale PD1 oder PD2 vorliegt.

Die Auswertung der ermittelten Drucksignale erfolgt über Vorrichtungen, die dem Fachmann aus dem Stand der Technik bekannt sind. Die Vorrichtung zum Auswerten der Messdaten kann z.B. eine CPU sein, welche Änderungen der gemessenen Drucksignale zu vorgegebenen Referenzwerten und/oder Änderungen zu vorher gemessenen Ausgangswerten berechnet. Die Auswertung kann in Form von absoluten und/oder relativen Änderungen, Differenzen in den gemessenen Werten, Änderungen in den Druckamplituden, z.B. der Höhe der Amplituden und/oder dem Frequenzspektrum erfolgen.

In einer bevorzugten Ausführungsform erfolgt die Auswertung der Drucksignale an einer zentralen Recheneinheit. Diese zentrale Recheneinheit umfasst eine CPU, einen Eingang für die Druckmesswerte und eine Anzeige für die Druckmesswerte und/oder die ermittelten Handlungsempfehlungen.

Die Effektivität der Blutbehandlung ist primär abhängig von vier Faktoren: der Behandlungszeit, dem Blutfluss, der Clearance und dem Dialysatfluss. Besonders die ausreichend lange Behandlungszeit muss gewährleistet sein und ist ein Hauptfaktor für eine erfolgreiche Behandlung. Zahlreiche Studien haben ergeben, je höher die verabreichte Dialysedosis, desto niedriger ist (über einen breiten Korrelationsbereich) auch die Patientensterblichkeit.

Ausfälle wegen Störungen im Betrieb akkumulieren sich schnell zu mehreren Sitzungen pro Jahr. Im Extremfall müssen Sitzungen sogar abgebrochen werden. Weitaus häufiger sind jedoch die Fälle, bei denen eine Störung nicht erkannt und daher auch nicht behoben wird, was zu einem suboptimalen Dialyseergebnis führt. Durch eine gezielte Behebung von Störungen, werden diese Auszeiten auf ein Minimum reduziert und können in manchen Fällen auch komplett vermieden werden, indem die richtige Maßnahme während des laufenden Betriebes eingeleitet wird. Die Suche nach dem Grund der Störung entfällt weitestgehend und gibt damit auch dem Patienten ein erhöhtes Gefühl der Sicherheit. Infolge dessen wird die Dialyseeffizienz gesteigert und auch die Wirtschaftlichkeit der Dialyse erhöht.

Der Begriff "*Flusseigenschaften*", wie hierin verwendet, bezieht sich auf die Gesamtheit der Eigenschaften der jeweils fließenden Flüssigkeit. Von besonderem Interesse sind die dynamische Viskosität, die Flussgeschwindigkeit, das Flussvolumen, das Strömungsprofil, osmotischer Druck, die Oberflächenspannung sowie die von den verwendeten Pumpen sowie den aktiven Betriebselementen wie elektrischen Vorrichtungen und passiven Betriebselementen wie dem Schlauchsystem und dem Dialysator erzeugten Veränderungen und Artefakte.

Der Begriff "*Verhältnis*", wie hierin verwendet, ist nicht zwingend auf den Quotienten aus zwei Größen beschränkt, sondern kann auch die Differenz oder jegliche andere Kennzahl, mit der sich das "*Verhältnis*" zwischen zwei Größen ausdrücken lässt, umfassen.

Die Analyse des Frequenzspektrums einzelner Drucksignale hat weiterhin gezeigt, dass eine Änderung der Permeabilität sich auf die Amplituden der harmonischen Frequenzen auswirkt. Gleiches gilt für Flussänderungen in Blutflussrichtung.

In einer bevorzugten Ausführungsform wird das Frequenzspektrum einzelner Signale sowie die relative Änderung in Bezug auf ein zweites Signal ermittelt. Durch Auswertung der beiden Frequenzspektren kann eine Aussage über die Flusseigenschaften in Blutflussrichtung, Dialysatflussrichtung oder in Transmembranrichtung getroffen werden, wobei Blutflussrichtung und Transmembranrichtung bevorzugt sind.

In einer weiteren Ausführungsform werden die Drucksignale einer Blutpumpe zur erfindungsgemäßen Differenzierung von Systemänderungen ermittelt.

In einer weiteren Ausführungsform werden die Drucksignale einer Bilanzkammer zur erfindungsgemäßen Differenzierung von Systemänderungen ermittelt.

In einer weiteren Ausführungsform erhöht das Verfahren zur Messung von Drucksignalen in einem Blutbehandlungssystem die Dialyseeffizienz und Wirtschaftlichkeit der Dialyse durch Differenzierung zwischen Systemänderungen, die in Blutflussrichtung oder in Transmembranrichtung auftreten.

Bei allen hierin beschriebenen Verfahren ist es bevorzugt, mehr als zwei Drucksignale und insbesondere 4 Drucksignale zeitgleich oder zeitversetzt an den Ein- und Auslässen des Tangentialflussfilters TFF zu messen, so wie durch die Drucksensoren [PB1], [PB2], [PD1] und [PD2] vorgenommen.

Die Erfindung umfasst weiterhin auch eine Vorrichtung zum Messen von Drucksignalen in einer Vorrichtung zur Behandlung von Blut, welche die Dialyseeffizienz und Wirtschaftlichkeit der Dialyse steigert, durch Differenzierung zwischen Systemänderungen, die in Blutflussrichtung oder in Transmembranrichtung auftreten, umfassend mindestens zwei Drucksensoren zur Messung von Drucksignalen.

Sämtliche zuvor beschriebene Ausführungsformen und Vorteile beziehen sich vorteilhafterweise auch auf das Verfahren und die Vorrichtung zur Messung von Drucksignalen in einem Blutbehandlungssystem, welches die Dialyseeffizienz und Wirtschaftlichkeit der Dialyse steigert, durch Differenzierung zwischen Systemänderungen, die in Blutflussrichtung oder in Transmembranrichtung auftreten.

Die Zusammensetzung der Drucksignale ist für das erfindungsgemäße Verfahren nicht erheblich. Erfindungsgemäß können Drucksignale aus einzelnen Quellen genutzt werden, aber auch Drucksignale, die die Summe aus einer Vielzahl von Quellen darstellen.

In einigen Ausführungsformen kann es vorteilhaft sein, wenn aus der Summe der Drucksignale nur eines ermittelt wird bzw. aus der Summe der Drucksignale nur eines herausgefiltert wird, um einen gültigen Wert für die übrigen Drucksignale zu ermitteln. Dies kann z.B. der Fall sein, wenn ein sehr unregelmäßiges Drucksignal die Messung der anderen überlagern würde oder wenn ein bestimmtes Drucksignal sich aufgrund seiner Eigenschaften besonders für die Messung eignet. Solche Einrichtungen zur Korrektur der Drucksignale sind aus dem Stand der Technik hinreichend bekannt.

Ferner wird die Aufgabe der vorliegenden Erfindung durch das Verfahren nach Anspruch 1 gelöst.

In einer bevorzugten Ausführungsform sind blutseitig ein Drucksensor [PB1] zwischen einer Pumpe P und einem Filter TFF und ein weiterer Drucksensor [PB2] zwischen dem Filter TFF und dem Patienten ☺ angebracht und dialysatseitig ein Drucksensor [PD2] hinter dem Ablauf des Filters TFF und ein weiterer Drucksensor [PD1] vor dem Zulauf zum Filter TFF angebracht.

In einer weiteren Ausführungsform umfasst die erfindungsgemäße Vorrichtung weiterhin eine Vorrichtung zum Auswerten der Messdaten. Dies kann eine CPU sein, welche Änderungen der gemessenen Drucksignale zu vorgegebenen Referenzwerten und/oder Änderungen zu vorher gemessenen Ausgangswerten berechnet.

Eine bevorzugte Ausführungsform umfasst die Vorrichtung zur Behandlung von Blut einen Tangentialflussfilter TFF, eine Pumpe P, und mindestens zwei Drucksensoren ([PB1], [PB2] oder [PD1], [PD2] oder [PB1], [PD1] oder [PB1], [PD2] oder [PB2], [PD1]oder [PB2], [PD2]), wobei die Drucksensoren ([PB1], [PB2] oder [PD1], [PD2] oder [PB1], [PD1] oder [PB1], [PD2] oder [PB2], [PD1]oder [PB2], [PD2]) dem Tangentialflussfilter TFF unmittelbar vorgeschaltet und/oder unmittelbar nachgeschaltet sind.

In einer weiter bevorzugten Ausführungsform umfasst die Vorrichtung zur Behandlung von Blut einen Tangentialflussfilter TFF, eine Pumpe P, und mindestens drei Drucksensoren ([PB1], [PB2], [PD1] oder [PB1], [PB2], [PD2] oder [PD1], [PD2], [PB1] oder [PD1], [PD2], [PB2]), wobei die Drucksensoren ([PB1], [PB2], [PD1] oder [PB1], [PB2], [PD2] oder [PD1], [PD2], [PB1] oder [PD1], [PD2], [PB2]) dem Tangentialflussfilter TFF unmittelbar vorgeschaltet und/oder unmittelbar nachgeschaltet sind.

Noch bevorzugter ist eine Vorrichtung zur Behandlung von Blut umfassend einen Tangentialflussfilter TFF, eine Pumpe P, und vier Drucksensoren [PB1], [PB2], [PD1] und [PD2], wobei die Drucksensoren [PB1], [PD1] dem Tangentialflussfilter TFF unmittelbar vorgeschaltet und die Drucksensoren [PB2], [PD2] dem Tangentialflussfilter TFF unmittelbar nachgeschaltet sind.

Wenn auf mindestens ein Drucksignal zurückgegriffen wird, das an mehreren Zeitpunkten nacheinander erfasst werden soll, um miteinander in Form eines Quotienten oder einer Differenz in Bezug gesetzt zu werden, und in einem zweiten Berechnungsschritt diese Sensor-spezifischen Quotienten oder Differenzen mit anderen Sensor-spezifischen Quotienten oder Differenzen in Bezug gesetzt werden, stellen sich die Verfahrensschritte wie folgt dar (Verfahren 2):

a) Zeitversetzte Messung von jeweils mindestens zwei Drucksignalen an mindestens zwei Drucksensoren, wobei die Drucksignale aus der Gruppe bestehend aus PB1, PB2, PD1 und PD2 ausgewählt werden, die Drucksensoren aus der Gruppe bestehend aus [PB1], [PB2], [PD1] und [PD2] ausgewählt werden,

[PB1] den Drucksensor im Blutkreislauf vor dem Bluteinlass in den Tangentialflussfilter TFF und PB1 das an [PB1] gemessene Drucksignal bezeichnet,

[PB2] den Drucksensor im Blutkreislauf nach dem Blutauslass aus dem Tangentialflussfilter TFF und PB2 das an [PB2] gemessene Drucksignal bezeichnet,

[PD1] den Drucksensor im Dialysatkreislauf vor dem Dialysateinlass in den Tangentialflussfilter TFF und PD1 das an [PD1] gemessene Drucksignal bezeichnet, und

[PD2] den Drucksensor im Dialysatkreislauf nach dem Dialysatauslass aus dem Tangentialflussfilter TFF und PD2 das an [PD2] gemessene Drucksignal bezeichnet;

b) Berechnung von mindestens zwei Quotienten und/oder Differenzen aus den gemäß Schritt a) an demselben Drucksensor gemessen zeitversetzten Drucksignalen in einer zentralen Recheneinheit, wobei die derart berechneten Quotienten gemäß dem Drucksensor, an dem die Messung durchgeführt wurde, als Q[PB1], Q[PB2], Q[PD1] und Q[PD2] und die Differenzen als Δ[PB1], Δ[PB2], Δ[PD1] und Δ[PD2] bezeichnet werden;

c) Berechnung mindestens eines Quotienten und/oder mindestens einer Differenz in der zentralen Recheneinheit aus den gemäß Schritt b) berechneten Quotienten und/oder Differenzen,

wobei die Quotienten und Differenzen aus der Gruppe bestehend aus (Q[PB1], Q[PB2]) und/oder (Q[PB1], Q[PD1]) und/oder (Q[PB1], Q[PD2]) und/oder (Q[PB2], Q[PD1]) und/oder (Q[PB2], Q[PD2]) und/oder (Q[PD1], Q[PD2]) und/oder (Δ[PB1], Δ[PB2]) und/oder (Δ[PB1], Δ[PD1]) und/oder (Δ[PB1], Δ[PD2]) und/oder (Δ[PB2], Δ[PD1]) und/oder (Δ[PB2], Δ[PD2]) und/oder (Δ[PD1], Δ[PD2]) und/oder (Q[PB1], (Δ[PB1]) und/oder (Q[PB1], (Δ[PB2]) und/oder (Q[PB1], (Δ[PD1]) und/oder (Q[PB1], (Δ[PD2]) und/oder (Q[PB2], (Δ[PB1]) und/oder (Q[PB2], (Δ[PB2]) und/oder (Q[PB2], (Δ[PD1]) und/oder (Q[PB2], (Δ[PD2]) und/oder (Q[PD1], (Δ[PB1]) und/oder (Q[PD1], (Δ[PB2]) und/oder (Q[PD1], (Δ[PD1]) und/oder (Q[PD1], (Δ[PD2]) und/oder (Q[PD2], (Δ[PB1]) und/oder (Q[PD2], (Δ[PB2]) und/oder (Q[PD2], (Δ[PD1]) und/oder (Q[PD2], (Δ[PD2]) und/oder (Q[PB1], Q[PBM]) und/oder (Q[PB2], Q[PBM]) und/oder (Q[PD1], Q[PBM]) und/oder (Q[PD2], Q[PBM]) und/oder (Δ[PB1], Q[PBM]) und/oder (Δ[PB2], Q[PBM]) und/oder (Δ[PD1], Δ[PBM]) und/oder (Δ[PD2], Q[PBM])

und/oder (Q[PB1], Q[PDM]) und/oder (Q[PB2], Q[PDM]) und/oder (Q[PD1], Q[PDM]) und/oder (Q[PD2], Q[PDM]) und/oder (Δ[PB1], Q[PDM]) und/oder (Δ[PB2], Q[PDM]) und/oder (Δ[PD1], Q[PDM]) und/oder (Δ[PD2], Q[PDM])

und/oder (Q[PB1], Δ[PBM]) und/oder (Q[PB2], Δ[PBM]) und/oder (Q[PD1], Δ[PBM]) und/oder (Q[PD2], Δ[PBM]) und/oder (Δ[PB1], Δ[PBM]) und/oder (Δ[PB2], A[PBM]) und/oder (Δ[PD1], Δ[PBM]) und/oder (Δ[PD2], Δ[PBM])

und/oder (Q[PB1], Δ[PDM]) und/oder (Q[PB2], Δ[PDM]) und/oder (Q[PD1], Δ[PDM]) und/oder (Q[PD2], Δ[PDM]) und/oder (Δ[PB1], Δ[PDM]) und/oder (Δ[PB2], Δ[PDM]) und/oder (Δ[PD1], Δ[PDM]) und/oder (Δ[PD2], Δ[PDM])

und/oder (Q[PBM], Q[PDM]) und/oder (Q[PBM], Δ[PBM]) und/oder (Q[PBM], Δ[PDM]) und/oder (Q[PDM], Δ[PBM]) und/oder (Q[PDM], Δ[PDM]) und/oder (Δ[PBM], Δ[PDM]) ausgewählt werden und

wobei Q[PBM] den Mittelwert aus Q[PB1] und Q[PB2], Q[PDM] den Mittelwert aus Q[PD1] und Q[PD2], Δ[PBM] den Mittelwert aus Δ[PB1] und Δ[PB2] bezeichnet, Δ[PDM] den Mittelwert aus Δ[PD1] und Δ[PD2] bezeichnet;

d) Mindestens einmaliges zeitversetztes Wiederholen der Schritte a), b) und c), wobei an denselben mindestens zwei Drucksensoren wie in der vorangegangenen Messung gemessen wird und aus den gemessenen Drucksignalen dieselben Quotienten und/oder Differenzen berechnet werden;

e) Erstellen mindestens eines zeitlichen Trends aus den gemessenen Drucksignalen ausgewählt aus den in c) gelisteten Gruppen;

f) Bewerten des mindestens einen zeitlichen Trends, ob eine Änderung des mindestens einen zeitlichen Trends über einen Toleranzbereich hinaus eingetreten ist;

g) Erstellen eines Musters für die Bewertung des mindestens einen zeitlichen Trends;

h) Zuordnung des Musters zu einem Störungsbild und

i) Anzeigen der Störung auf einer Anzeigevorrichtung der zentralen Recheneinheit.

Diese Ausführungsform ist lediglich eine Ergänzung des zuvor beschriebenen Hauptverfahrens dieser Erfindung. Hierbei wird dem Berechnungsschritt zuvor beschriebenen Hauptverfahren lediglich der Rechenschritt der zeitversetzten Messung an mindestens zwei Drucksensoren vorausgestellt. Mit diesem alternativen Verfahren lassen sich einige Störungen im Dialyseablauf, die bereits durch das erste Verfahren detektiert und lokalisiert werden können, im Zweifelsfall schärfer abbilden.

Der Übersichtlichkeit halber werden die in diesem zusätzlichen Rechenschritt berechneten Quotienten gemäß dem Drucksensor, an dem sie ermittelt wurden, als Q[PB1], Q[PB2], Q[PD1] und Q[PD2] und die Differenzen als Δ[PB1], Δ[PB2], Δ[PD1] und Δ[PD2] bezeichnet.

Es versteht sich von selbst, daß nur die Kombinationen berechnet werden können, die sich aus der Vorwahl der Anzahl der Messwiederholungen und der Anzahl der Drucksensoren logisch möglich sind, um das Verfahren erfindungsgemäß durchzuführen.

Optional kann dieses Verfahren zusätzlich den folgenden Schritt umfassen:

j) Behebung der Störung des Flusswiderstandes in transmembraner Richtung, Dialysatflussrichtung oder Blutflussrichtung oder Angabe einer Möglichkeit zur Behebung der Störung.

Bezüglich der Schritte i) und j) gilt in Analogie das gleiche wie oben zum Verfahren 1 ausgeführt.

Auf der zentralen Recheneinheit können bereits Toleranzbereiche für die Bewertung der zeitlichen Trends der gemessenen Drucksignale und/oder der Quotienten und/oder der Differenzen aus den Drucksignalen hinterlegt sein. Diese zeitlichen Trends der gemessenen Drucksignale und/oder Quotienten und/oder der Differenzen der Drucksignale können aus einer oder mehrerer vorheriger Sitzungen von einem bestimmten Patienten mit diesem spezifischen Dialysegerät stammen. Es können gegebenenfalls aber auch Messwerte dieses Patienten von anderen Dialysegeräten verwendet werden, gleichfalls Messwerte von diesem spezifischen Dialysegerät, die nicht patientenspezifisch sind. Es kann sich aber auch aus der Literatur bekannte Richtwerte oder gerätetypische Spezifikationen des Herstellers des Dialysegerätes handeln.

Mit "unmittelbar" ist gemeint, dass zwischen dem Drucksensor und dem genannten Bauteil kein weiteres Bauteil liegt. Die tatsächliche Entfernung zwischen dem Drucksensor und dem genannten Bauteil ist hierbei nicht ausschlaggebend, sondern nur, dass der Drucksensor und das genannte Bauteil nicht durch ein weiteres dazwischenliegendes Bauteil getrennt sind. Erfindungsgemäß liegen zwei Drucksensoren nie unmittelbar hintereinander, also ohne ein weiteres Bauteil zwischen den Drucksensoren, Zudem verwendet die vorliegende Erfindung keinen Drucksensor im Blutkreislauf zwischen Patient ☺ und Pumpe P, weil derartige Drucksensoren zur Überwachung des Patienten dienen und zur Überwachung von Systemänderungen in dem Tangentialflussfilters TFF nicht geeignet sind.

In weiteren bevorzugten Ausführungsformen kann jedoch blutseitig ein Blasenfänger zwischen dem Drucksensor und dem Bauteil angeordnet sein, und/oder auf der Dialysatseite ein Filter. Das zuvor beschriebene Grundprinzip wird aber dadurch nicht in Frage gestellt.

Die Begriffe "vorgeschaltet" und "nachgeschaltet" sind mit Bezug auf die Flussrichtung zu verstehen. Ist ein Drucksensor "vorgeschaltet", so liegt er in Flussrichtung vor dem Bauteil, d.h. das Blut oder die Dialyseflüssigkeit durchläuft zuerst den Drucksensor und dann das Bauteil. Ist ein Drucksensor "nachgeschaltet", dann liegt er in Flussrichtung hinter dem Bauteil, d.h. das Blut oder die Dialyseflüssigkeit durchläuft zuerst das Bauteil und dann den Drucksensor. Die Flussrichtung kann im Blutkreislauf und im Dialysekreislauf gegensätzlich zueinander sein.

Die erfindungsgemäßen Vorrichtungen zur Behandlung von Blut können weiterhin eine Ultrafiltrationspumpe UFP, ein Bilanzkammersystem BK oder eine Bilanzkammer BK und/oder eine Einheit zum Auswerten der gemessenen Drucksignale umfassen. Die Ultrafiltrationspumpe wird für die kontinuierlich geregelte Ultrafiltration benötigt und entnimmt dem geschlossenen System eine genau eingestellte Flüssigkeitsmenge. Dieselbe Menge, die dem geschlossenen Dialysatkreislauf entnommen wird, wird im Tangentialflussfilter TFF dem Blut mittels Unterdruck entzogen. Für die Bilanzierung der ein- und ausgehenden Flüsse sorgt die Bilanzkammer BK, somit wird gewährleistet, dass dem Patienten keine Flüssigkeit unbeabsichtigt entzogen oder zugeführt wird. Die Bilanzkammer kann dabei durch eine flexible Trennwand in zwei Kammerhälften unterteilt werden, die wechselseitig mit dem Ultrafiltrat befüllt werden, das dem Dialysatkreislauf entzogen wird, wobei der Inhalt der jeweils anderen Kammerhälfte verworfen wird. Optional umfasst die erfindungsgemäße Vorrichtung weiterhin eine Tropfkammer. Die Tropfkammer hilft, das Eindringen von Luft in die stromabwärtigen Schläuche zu verhindern, indem eine Flüssigkeitsschicht als eine Luftsperre am Boden der Tropfkammer fungiert.

Die Drucksensoren [PB1], [PB2], [PD1] und [PD2] sind vorteilhafterweise dadurch charakterisiert, dass sie eine Abtastrate von mindestens 20 Hz haben. Eine Abtastrate von 20 Hz bedeutet, dass eine Druckmessung pro Drucksensor 20-mal pro Sekunde erfolgt.

Die Begriffe "dialysatseitig" und "blutseitig" beschreiben die beiden Kreisläufe, die bei der Tangentialflussfiltration, in der Regel im Gegenstromprinzip, aber ggf. auch parallel zum Blutstrom, aneinander vorbeigeführt werden. In die Hohlfasern der Filtermembran werden über einen ersten Flüssigkeitskreislauf Blut/Plasma zugeführt, das sie der Länge nach durchströmt. Durch einen zweiten Flüssigkeitskreislauf wird die Dialyseflüssigkeit auf der Außenseite der Hohlfasern zugeführt. Beide Kreisläufe sind voneinander getrennt und stehen nur über die Filtermembran miteinander in Kontakt.

Der Begriff "Toleranzbereich" bezieht sich auf einen Bereich um einen idealtypisch angenommenen Erwartungswert eines Drucksignals an einem gegebenen Druckmesspunkt zu einem gegebenen Zeitpunkt im Laufe der Behandlung, innerhalb dessen Abweichungen von dem Erwartungswert toleriert werden. Dieser Toleranzbereich wird vor dem Beginn einer Dialysesitzung festgelegt, kann aber auch als fabrikatsspezifisch bereits vom Hersteller festgelegt worden sein. Hierbei kann es sich um Absolutwerte handeln oder aber um einen absoluten oder prozentualen Bereich um einen Erwartungswert herum, wobei der Erwartungswert erst im Laufe der Dialysesitzung ermittelt, aus einer auf der zentralen Recheneinheit hinterlegten Datei abgerufen oder zuvor eingegeben wird. Messwerte, die in ein derart festgelegten Toleranzbereich fallen, werden als "gleichbleibend" beschrieben.

Der Begriff "Muster", wie hier verwendet, bezieht sich auf eine mathematische, elektronische und/oder graphische Darstellungsform des Trends mehrerer Quotienten und/oder Differenzen und/oder absoluter Werte von Drucksignalen über die Zeit. Aus diesem Muster läßt sich unmittelbar die Bewertung "schnell steigend", "steigend", "gleichbleibend", "fallend" oder "schnell fallend" ableiten.

Der Begriff "jedes mögliche Muster" meint, daß nur einige der besagten Muster durch Störungen im Betrieb an einer Blutbehandlungseinheit auftreten können. Andere Muster können aus logischen Gründen nicht auftreten oder wären einem nicht erfindungsrelevanten Fehler im System geschuldet, wie beispielsweise einem defekten Druckmessgerät. Solche Konstellationen sollen durch den Begriff "jedes mögliche Muster" nicht abgedeckt sein und bedürfen im Rahmen der vorliegenden Erfindung keiner näheren Betrachtung.

Der Begriff "Mustertabelle" bezieht sich auf ein mathematisches, elektronisches und/oder graphisches Register oder eine entsprechende Matrix, worin für jede denkbare mögliche Fallkombination der Bewertungen steigend, gleichbleibend oder fallend für die bis zu vier Messpunkte eine Angabe über den Befund der Störung und eine vorgeschlagene Maßnahme zu deren Behebung hinterlegt ist. In bevorzugten Ausführungsformen ist in der Mustertabelle nicht nur eine qualitative Angabe über die wahrscheinliche Störung angeführt, sondern auch ein Maß oder eine Formel, um aus dem Messwerten der Drucksignale zu berechnen, in welchem Umfang die variablen Parameter an der Blutbehandlungseinheit nachzuregeln sind. In weiteren bevorzugten Ausführungsformen wird nach einem Abgleich des aus den aktuellen Drucksignalen ermittelten Musters mit der Mustertabelle die empfohlene qualitative und/oder quantitative Anpassung automatisch ausgeführt. Hierzu bedarf es einer Übermittlung des Maßes der Anpassung von der zentralen Recheneinheit an das mindestens eine Stellglied an der Blutbehandlungseinheit, um die ermittelte Anpassungsmaßnahme umzusetzen.

Die Behebung einer Störung des Flusswiderstandes in transmembraner Richtung kann in einer Regulation des Blutflusses, einer Regulation des Dialysatflusses, einer abgestimmten Regelung von Blut- und Dialysatfluss, einem transmembranen Spülvorgang, einem blutseitigen Spülvorgang, einer Veränderung der Behandlungsdauer, einer Regulation der Ultrafiltrationsrate, einer Kombination der vorigen oder einem Austausch des Filtermoduls bestehen. Der Austausch des Filtermoduls kann nur bei Unterbrechung oder nach Beendigung der Dialysesitzung erfolgen. Alle anderen Maßnahmen können online während der Dialysesitzung eingeleitet werden.

Die Behebung einer Störung des Flusswiderstandes in Blutflussrichtung kann in einer Regulation des Blutflusses, einer Regulation des Dialysatflusses, einer abgestimmten Regelung von Blut- und Dialysatfluss, einer Blutverdünnung vor der Filter (predilution), einem blutseitigen Spülen, einem transmembranen Spülen, einer Zugabe von Antikoagulanzien, einer Veränderung der Behandlungsdauer, einer Regulation der Ultrafiltrationsrate, einer Kombination der vorigen oder einem Austausch von mindestens einem Schlauch oder einem Schlauchsystem bestehen. Der Austausch eines Schlauches oder eines Schlauchsystems kann nur bei Unterbrechung oder nach Beendigung der Dialysesitzung erfolgen. Mitunter kann eine Störung aber auch im Umknicken oder Kollabieren eines oder mehrerer Schläuche bestehen. Diese Fehler sind je nach Bauart der Dialysemaschine meist relativ leicht zu beheben. Wie im vorherigen Fall können aber die meisten anderen Maßnahmen online während der Dialysesitzung eingeleitet werden.

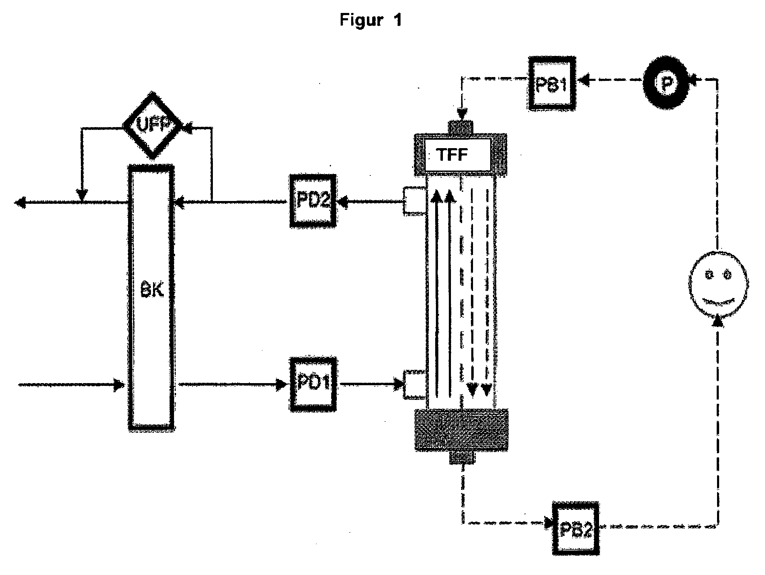

Ein schematisches Flusssystem mit den folgenden Komponenten ist in Fig. 1 abgebildet: Eine Pumpe P, vorzugsweise eine peristaltische Pumpe, erzeugt den gewünschten Fluss im extrakorporalen Kreislauf. Im Blutkreislauf, mit gestrichelten Linien dargestellt, durchläuft das Blut vom Patienten ☺ aus zunächst die Pumpe P, dann den ersten blutseitigen Drucksensor [PB1], den Tangentialflussfilter TFF und bevor es in den Patienten ☺ zurückfließt noch einen weiteren Drucksensor [PB2]. Im Gegenstromprinzip wird Dialyseflüssigkeit durch den Filter TFF gepumpt. Im Dialysekreislauf, mit durchgehenden Linien dargestellt, befindet sich in Flussrichtung vor dem Filter TFF der erste dialysatseitige Drucksensor [PD1] und hinter dem Filter TFF der zweite Drucksensor [PD2]. Für die Bilanzierung der ein- und ausgehenden Flüsse sorgt die Bilanzkammer BK, somit wird gewährleistet, dass dem Patienten ☺ keine Flüssigkeit unbeabsichtigt entzogen oder zugeführt wird. Der für die Therapie verordnete Gewichtsverlust wird durch die Ultrafiltrationspumpe UFP erzeugt, die das Bilanzkammersystem BK umgeht.

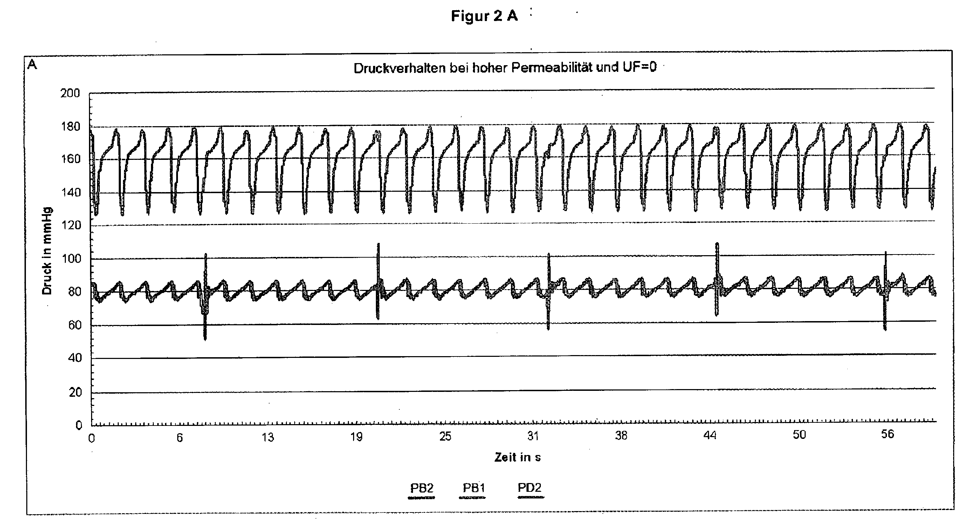

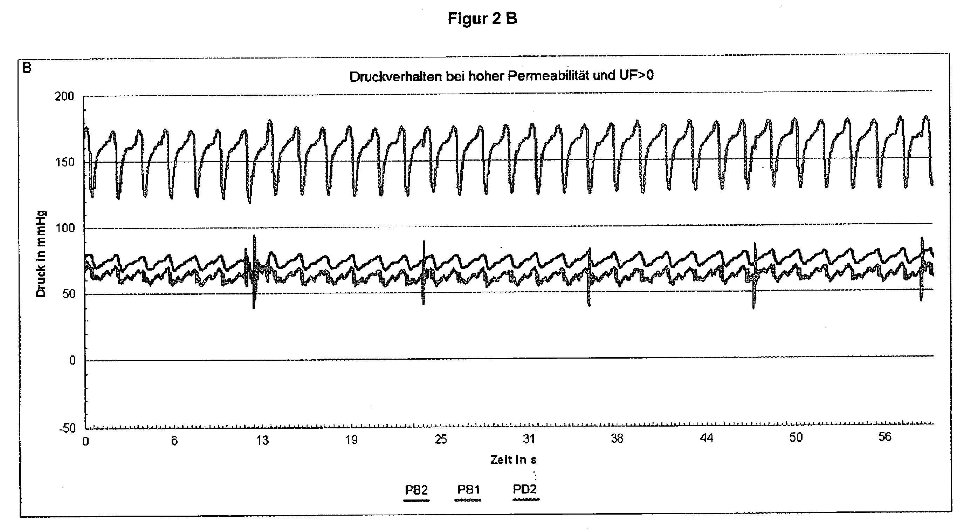

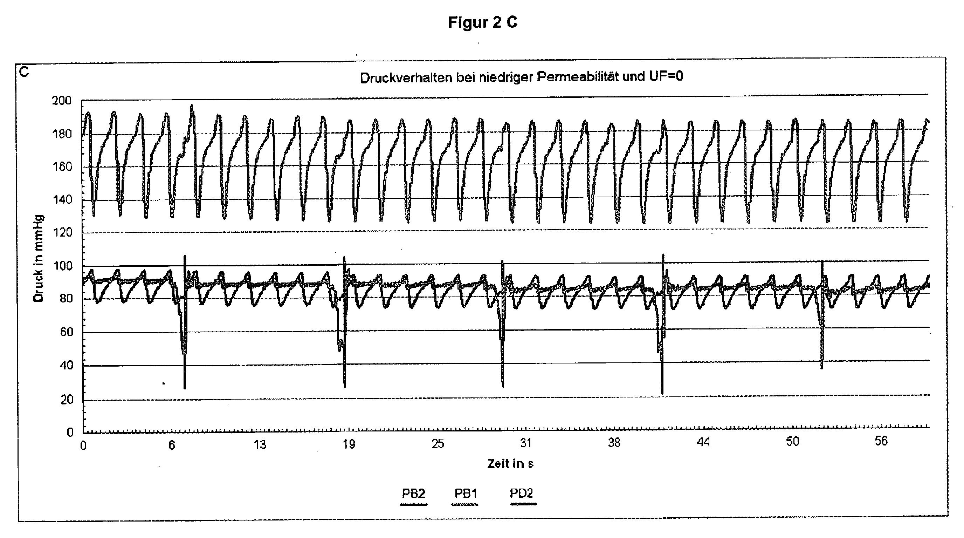

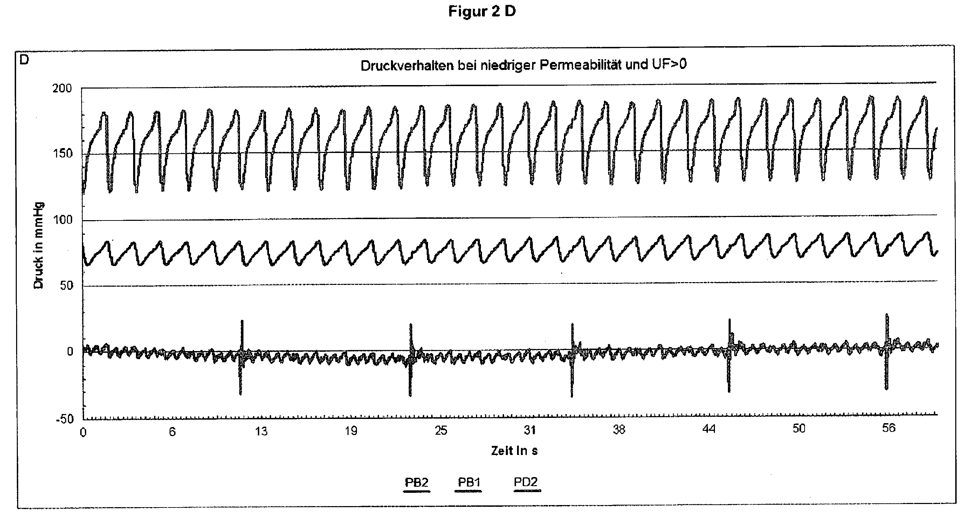

Der Druckverlauf der Drücke PB1, PB2 und PD2 unter verschiedenen Behandlungsbedingungen läßt sich anhand von Fig. 2 nachvollziehen. A und B sind die Druckverläufe eines Dialysators mit hoher Permeabilität bei UF=0 (A) und UF>0 (B). C und D sind die Druckverläufe eines Dialysators mit niedrigerer Permeabilität bei UF=0 (C) und UF>0 (D). Die Dialysatorgeometrie (Faserlänge, Faserinnendurchmesser und Faseranzahl) bleibt konstant. Man erkennt in A das Drucksignal PB1 vor dem Dialysator. Es ist in Amplitude und Absolutwert das stärkste Signal. PB2 und PD2 liegen in ihrer Amplitude und dem Absolutwert darunter. Man erkennt auch, daß die Signale zwar phasenverschoben gegenüber PB1 sind, untereinander aber keine Phasenverschiebung aufweisen.

Vergleicht man die Signale aus A mit denen aus B, erkennt man, daß sich die Phase nicht verändert hat. Die Lage der Drücke PB1 und PB2 ist unverändert. Der Absolutwert von PD2 ist jedoch niedriger und die Amplitude von PB1 ist verringert, was durch die Erhöhung der UF-Rate zu erklären ist. Die dem Drucksignal PD2 aufgeprägte Störung wird in diesem Fall durch die UF-Pumpe, eine Zahnradpumpe, erzeugt.

Vergleicht man C und D miteinander, so erkennt man, daß die Erhöhung der UF-Rate die gleichen Auswirkungen hat. Die in PD2 speziell bei UF>0 auftretenden hochfrequenten Pulse sind wiederum durch die UF-Pumpe verursacht.

Vergleicht man die unterschiedlichen Permeabilitäten bei gleichen UF-Raten, erkennt man, daß die Amplituden auf der Blutseite (bei PB1 und PB2) bei geringerer Permeabilität größer sind. Das liegt daran, daß der fehlende Übertrag auf die Dialysatseite, der durch einen erhöhten transmembranen Flusswiderstand verursacht wird, durch eine Erhöhung der blutseitigen Pulsamplituden kompensiert werden muss.

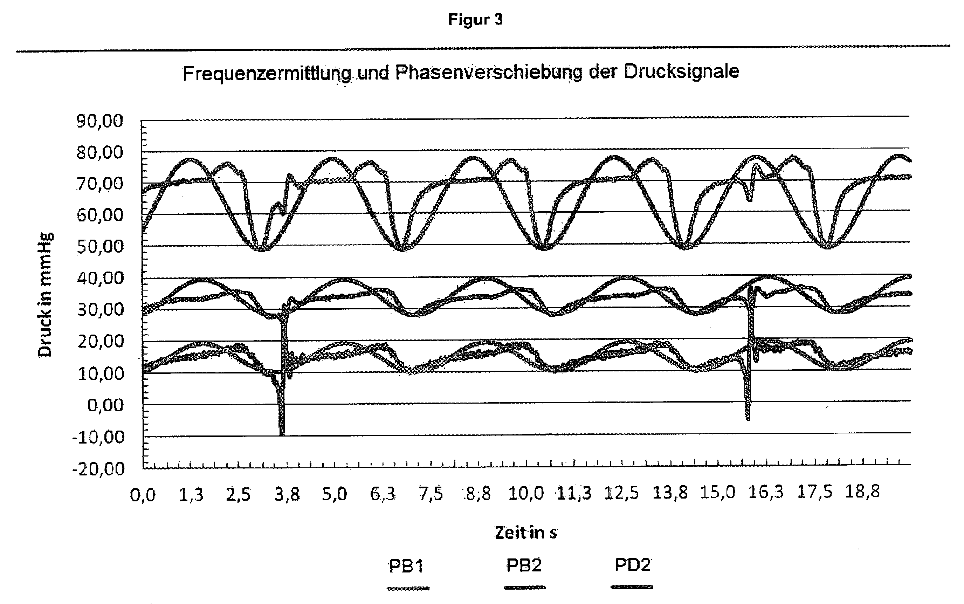

Der Druckverlauf für die Drücke PB1, PB2 und PD1 bei einem Blutfluss von 100 ml/min ist in Fig. 3 dargestellt. Über die Druckkurven wurde jeweils eine Sinusfunktion gelegt, deren Minima sich mit den Minima des Drucksignals decken. So wird die Periodizität, Grundlage der Berechnung der Phasenverschiebung sowie der Frequenzanalyse verdeutlicht.

Die Funktion, mit der die Sinusfunktion angepasst wurde, ist:

$$G = A \cdot \sin(2 \cdot \pi \cdot f \cdot t + \varphi) + B$$

(A: Amplitude in mm Hg, *f*: Frequenz in Hz, t: Zeit, $\varphi$: Anfangsphase in rad, B: Offset).

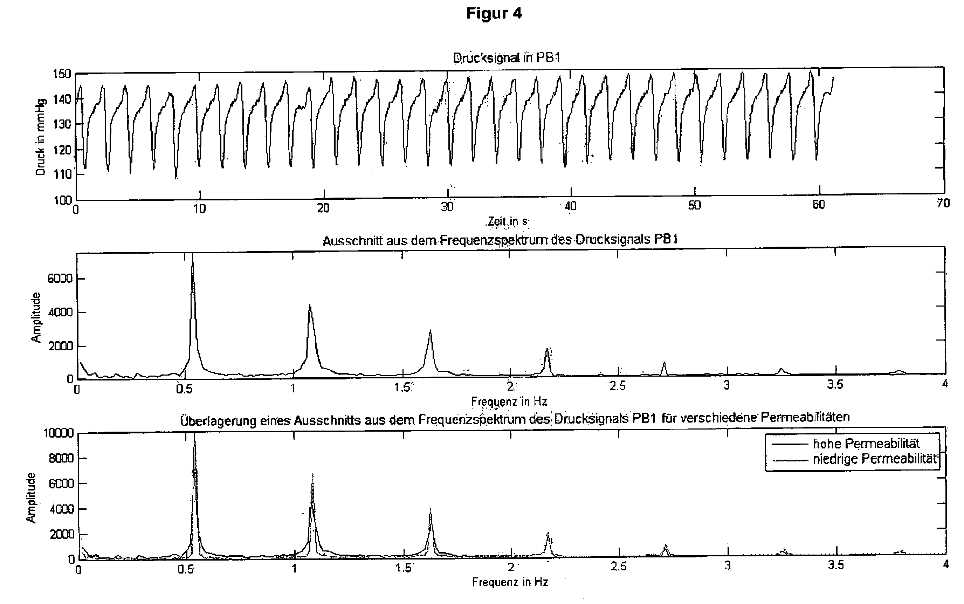

Eine Frequenzanalyse läßt sich beispielhaft an einem Drucksignal von PB1 zeigen (Fig. 4). Der Verlauf des Drucksignals entspricht demjenigen aus den Abbildungen 2 A-D. In der oberen Grafik ist der Zeitverlauf über 60 s dargestellt. In der mittleren Grafik ist ein Ausschnitt des zugehörigen Frequenzspektrums aufgetragen. Die untere Grafik zeigt den Vergleich zwischen den Amplituden der Spektra des Drucksignals PBE für unterschiedliche Permeabilitäten. Die Verringerung der Permeabilität erhöht die Frequenzamplitude des blutseitigen Druckaufnehmers [PB1]. Dies wird besonders deutlich für die Grundschwingung sowie die ersten höheren Ordnungen.

Die Erfindung bezieht sich ebenfalls auf eine Vorrichtung, die in der Lage ist, die zuvor beschriebenen Messverfahren und Anpassungsmaßnahmen durchzuführen. Eine solche Vorrichtung ist ein Blutbehandlungssystem.

Ein "Blutbehandlungssystem", wie hierin verwendet, umfasst eine Blutbehandlungseinheit, deren Kernstück ein Tangentialflussfilter TFF ist, mindestens zwei Drucksensoren, die aus der Gruppe bestehend aus [PB1], [PB2], [PD1] und [PD2] ausgewählt werden, und eine zentralen Recheneinheit, wobei die mindestens zwei Drucksensoren mit der zentralen Recheneinheit zur Übertragung der Messwerte verbunden sind und die zentrale Recheneinheit befähigt ist, die eingehenden Messwerte derart auszuwerten und anzuzeigen, daß eine Unterscheidung von Flusswiderstandsänderungen in transmembraner Richtung, Dialysatflussrichtung und Blutflussrichtung in dem Blutbehandlungssystem ermöglicht wird.

Somit bezieht sich die Erfindung auch auf eine Vorrichtung, die Folgendes umfasst: Eine Blutbehandlungseinheit und mindestens zwei Drucksensoren, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilters TFF, [PB2] den Drucksensor im Blutkreislauf nach dem Blutauslass aus dem Tangentialflussfilter TFF, [PD1] den Drucksensor im Dialysatkreislauf vor dem Dialysateinlass in den Tangentialflussfilter TFF und [PD2] den Drucksensor im Dialysatkreislauf nach dem Dialysatauslass aus dem Tangentialflussfilter TFF bezeichnet und einer zentralen Recheneinheit besteht, wobei die Vorrichtung geeignet ist ein Verfahren zur Unterscheidung von Störungen des Flusswiderstandes in einer Blutbehandlungseinheit mit einem Tangentialflussfilter TFF auszuführen, das die folgenden Schritte umfasst:

a) Messung von mindestens zwei Drucksignalen ausgewählt aus der Gruppe bestehend aus (PB1, PB2, PD1, PD2), die an mindestens zwei Drucksensoren gemessen werden, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;

b) Berechnung der Quotienten und/oder der Differenzen aus den gemäß Schritt a) gemessen Drucksignalen, wobei die Quotienten und/oder Differenzen berechnet werden aus den Drucksignalen ausgewählt aus der Gruppe bestehend aus (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM) in einer zentralen Recheneinheit, wobei mit PBM oder PDM der jeweilige Mittelwert aus (PB1, PB2) bzw. (PD1, PD2) bezeichnet wird;

c) Mindestens einmaliges zeitversetztes Wiederholen der Schritte a) und b), wobei an denselben mindestens zwei Drucksensoren wie in der vorangegangenen Messung gemessen wird und aus den gemessenen Drucksignalen dieselben Quotienten und/oder Differenzen berechnet werden;

d) Erstellen mindestens eines zeitlichen Trends aus den gemessenen Drucksignalen ausgewählt aus der Gruppe bestehend aus (PB1, PB2, PD1, PD2) und/oder den berechneten Differenzen oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM);

e) Bewerten des mindestens einen zeitlichen Trends, ob eine Änderung des mindestens einen zeitlichen Trends über einen Toleranzbereich hinaus eingetreten ist;

f) Erstellen eines Musters für die Bewertung des mindestens einen zeitlichen Trends;

g) Zuordnung des Musters zu einem Störungsbild und

h) Anzeigen der Störung auf einer Anzeigevorrichtung der zentralen Recheneinheit.

**EP 2 627 368 B1**

Weiterhin bezieht sich die Erfindung auch auf eine Vorrichtung, die Folgendes umfasst:

Eine Blutbehandlungseinheit und mindestens drei Drucksensoren, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilters TFF, [PB2] den Drucksensor im Blutkreislauf nach dem Blutauslass aus dem Tangentialflussfilter TFF, [PD1] den Drucksensor im Dialysatkreislauf vor dem Dialysateinlass in den Tangentialflussfilter TFF und [PD2] den Drucksensor im Dialysatkreislauf nach dem Dialysatauslass aus dem Tangentialflussfilter TFF bezeichnet und einer zentralen Recheneinheit besteht, wobei die Vorrichtung geeignet ist ein Verfahren zur Unterscheidung von Störungen des Flusswiderstandes in einer Blutbehandlungseinheit mit einem Tangentialflussfilter TFF auszuführen, das die folgenden Schritte umfasst:

a) Messung von mindestens drei Drucksignalen ausgewählt aus der Gruppe bestehend aus (PB1, PB2, PD1, PD2), die an mindestens drei Drucksensoren gemessen werden, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;
b) Berechnung der Quotienten und/oder der Differenzen aus den gemäß Schritt a) gemessen Drucksignalen, wobei die Quotienten und/oder Differenzen berechnet werden aus den Drucksignalen ausgewählt aus der Gruppe bestehend aus (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM) in einer zentralen Recheneinheit, wobei mit PBM oder PDM der jeweilige Mittelwert aus (PB1, PB2) bzw. (PD1, PD2) bezeichnet wird;
c) Mindestens einmaliges zeitversetztes Wiederholen der Schritte a) und b), wobei an denselben mindestens drei Drucksensoren wie in der vorangegangenen Messung gemessen wird und aus den gemessenen Drucksignalen dieselben Quotienten und/oder Differenzen berechnet werden;
d) Erstellen mindestens eines zeitlichen Trends aus den gemessenen Drucksignalen ausgewählt aus der Gruppe bestehend aus (PB1, PB2, PD1, PD2) und/oder den berechneten Differenzen und/oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM);
e) Bewerten des mindestens einen zeitlichen Trends, ob eine Änderung des mindestens einen zeitlichen Trends über einen Toleranzbereich hinaus eingetreten ist;
f) Erstellen eines Musters für die Bewertung des mindestens einen zeitlichen Trends;
g) Zuordnung des Musters zu einem Störungsbild und
h) Anzeigen der Störung auf einer Anzeigevorrichtung der zentralen Recheneinheit.

Weiterhin bezieht sich die Erfindung auch auf eine Vorrichtung, die Folgendes umfasst:

Eine Blutbehandlungseinheit und vier Drucksensoren [PB1], [PB2], [PD1] und [PD2], wobei [PB1] den Drucksensor im Blutkreislauf vor dem Bluteinlass in den Tangentialflussfilters TFF, [PB2] den Drucksensor im Blutkreislauf nach dem Blutauslass aus dem Tangentialflussfilter TFF, [PD1] den Drucksensor im Dialysatkreislauf vor dem Dialysateinlass in den Tangentialflussfilter TFF und [PD2] den Drucksensor im Dialysatkreislauf nach dem Dialysatauslass aus dem Tangentialflussfilter TFF bezeichnet und einer zentralen Recheneinheit besteht,
wobei die Vorrichtung geeignet ist ein Verfahren zur Unterscheidung von Störungen des Flusswiderstandes in einer Blutbehandlungseinheit mit einem Tangentialflussfilter TFF auszuführen, das die folgenden Schritte umfasst:

a) Messung von vier Drucksignalen (PB1, PB2, PD1, PD2), die an vier Drucksensoren gemessen werden ([PB1], [PB2], [PD1] und [PD2]), wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;

b) Berechnung der Quotienten und/oder der Differenzen aus den gemäß Schritt a) gemessen Drucksignalen, wobei die Quotienten und/oder Differenzen berechnet werden aus den Drucksignalen (PB1, PB2) und (PB1, PD1) und (PB1, PD2) und (PB2, PD1) und (PB2, PD2) und (PD1, PD2) und (PB1, PDM) und (PB2, PDM) und (PD1, PBM) und (PD2, PBM) und (PDM, PBM) in einer zentralen Recheneinheit, wobei mit PBM oder PDM der jeweilige Mittelwert aus (PB1, PB2) bzw. (PD1, PD2) bezeichnet wird;

c) Mindestens einmaliges zeitversetztes Wiederholen der Schritte a) und b), wobei an denselben vier Drucksensoren wie in der vorangegangenen Messung gemessen wird und aus den gemessenen Drucksignalen dieselben Quotienten und/oder Differenzen berechnet werden;

d) Erstellen der zeitlichen Trends aus den gemessenen Drucksignalen (PB1, PB2, PD1, PD2) und/oder den berechneten Differenzen und/oder Quotienten der Drucksignale (PB1, PB2) und (PB1, PD1) und (PB1, PD2) und (PB2, PD1) und (PB2, PD2) und (PD1, PD2) und (PB1, PDM) und (PB2, PDM) und (PD1, PBM) und (PD2, PBM) und (PDM, PBM);

e) Bewerten der zeitlichen Trends, ob eine Änderung der zeitlichen Trends über einen Toleranzbereich hinaus eingetreten ist;

f) Erstellen eines Musters für die Bewertung der zeitlichen Trends;

g) Zuordnung des Musters zu einem Störungsbild und

h) Anzeigen der Störung auf einer Anzeigevorrichtung der zentralen Recheneinheit.

In bevorzugten Ausführungsformen umfasst das besagte Blutbehandlungssystem zusätzlich eine Ultrafiltrationspumpe und/oder ein Bilanzkammersystem umfasst.

Erfindungsgemäß haben die Drucksensoren [PB1], [PB2], [PD1] und [PD2] bei diesem Blutbehandlungssystem jeweils eine Abtastrate von mindestens 20 Hz.

**Figurenbeschreibung**

| | |
|---|---|
| **Figur 1:** | Schema für ein erfindungstypisches Flusssystem |
| **Figuren 2 A-D:** | Druckverlauf der Drücke PB1, PB2 und PD2 unter verschiedenen Behandlungsbedingungen |

2A: Druckverhalten bei hoher Permeabilität und UF = 0
2B: Druckverhalten bei hoher Permeabilität und UF > 0
2C: Druckverhalten bei niedriger Permeabilität und UF = 0
2D: Druckverhalten bei niedriger Permeabilität und UF > 0

| | |
|---|---|
| **Figur 3:** | Druckverlauf für die Drücke PB1, PB2 und PD1 bei einem Blutfluss von 100 ml/min. Daran gefittete Sinusfuktion. |
| **Figur 4:** | Frequenzanalyse des Drucksignals PB1 |
| **Figur 5:** | Signal PD2 bei hohen Permeabilitäten (oben). Vergleich der Frequenzspektra bei einer hohen und einer verringerten Permeabilität (unten). |
| **Figur 6:** | Musterkonstellationen für auftretende Druckveränderungen (Absolutdrücke und Quotienten) |

+ steigt an
++ steigt schnell an
- fällt ab
-- fällt schnell ab
0 bleibt unverändert

| | |
|---|---|
| **Figur 7:** | Musterkonstellationen für auftretende Druckveränderungen (Absolutdrücke und Differenzen) |

+ steigt an
++ steigt schnell an
- fällt ab
-- fällt schnell ab

0 bleibt unverändert

**Beispiele**

**Beispiel 1:**

Analyse der Amplituden der Druckpulse:

Ein periodischer Druckverlauf wird in einzelne Druckpulse zerlegt und die Amplitude jedes Pulses aus dem Minimal- und Maximalwert bestimmt. Dazu werden die periodisch auftretenden Minima eines Druckverlaufes ermittelt. Als ein Druckpuls wird ein Abschnitt von einem Minimum bis zum folgenden betrachtet. Die Breite der periodischen Druckpulse ist durch die Struktur der Blutpumpe und durch den Blutfluss (BF) bestimmt. Die Pulsbreite ist für alle Drücke und Ultrafiltrationsraten (UF-Rate) bei festem BF gleich, die aber mit steigendem BF wachsen. Für jeden Puls werden der minimale und der maximale Druck ausgelesen. Die Amplitude ist durch die Differenz dieser Extremwerte gegeben. Die Verhältnisse $A_{PD1}/A_{PB1}$ und $A_{PD2}/A_{PB1}$ sowie $A_{PD1}/A_{PB2}$ und $A_{PD2}/A_{PB2}$ zeigen einen deutlichen Anstieg mit wachsender Faserpermeabilität. Deutlich zu erkennen ist dies in Figur 2. Mit wachsendem BF steigen die Druckpulse an, aber die Verhältnisse bleiben im wesentlichen konstant. Eine Erhöhung der UF-Rate hat die gleichen Auswirkungen auf das Druckverhalten wie oben beschrieben die Permeabilität. In beiden Fällen wird der transmembrane Impulsübertrag verbessert. Eine Veränderung des Widerstands in Blutflussrichtung schlägt sich wie zu erwarten in einer Verkleinerung des Verhältnisses der Amplituden aus $A_{PB2}/A_{PB1}$ nieder. Da die Dämpfung in Flussrichtung erhöht wird, fällt $A_{PB2}$. Wie zu erwarten steigen somit die Amplituden auf der Dialysatseite genauso wie die aufgeführten abhängigen Verhältnisse. Das gleiche Verhalten zeigt sich in umgekehrter Richtung, wenn auf der Dialysatseite Pulse erzeugt werden, beispielsweise durch ein Bilanzsystem zur Kompensation der Ultrafiltration. Somit können auch diese zur Charakterisierung des Systems verwendet werden, sowohl transmembran als auch in Dialysatflussrichtung.

**Beispiel 2:**

Analyse der Phasenverschiebung der Drucksignale

Die Druckverläufe lassen sich bezüglich der Positionen der Minima gut durch eine Sinusfunktion mit fester Frequenz und Phasenverschiebung approximieren. Die Frequenz ist für die jeweiligen Drücke identisch und wird durch den Blutfluss und die periodisch arbeitende Blutpumpe, in der Regel eine Schlauchpumpe bestimmt. Der Kehrwert der Frequenz entspricht der zeitlichen Breite eines einzelnen Druckpulses. Zwischen den einzelnen Druckverläufen liegt eine Phasenverschiebung vor, die unter anderem von der Permeabilität abhängig ist. Der Füllstand des Luftblasenfängers und die Länge des Schlauchsystems haben ebenfalls einen Einfluss und sollten somit während der Messungen konstant gehalten werden. Bei sinkender Permeabilität wird die Phasenverschiebung von PB1 zu PD1 und PD2 größer, während die von PB1 zu PB2 abnimmt.

Bei der Verwendung eines high-flux Dialysators (Dialysefilters) mit einer hydraulischen Permeabilität von 275 ml/(h m$^2$ mmHg) sind sowohl PB2 als auch PD2 um 0,53 rad gegenüber PB1 verschoben (siehe Figur 3). Wird die Permeabilität bis zu der von einem low-flux Dialysator (14 ml/(h m$^2$ mmHg)) verändert, ist PB2 gegen PB1 um 0,38 rad verschoben und PD2 um 0,65 rad phasenverschoben gegen PB1. Dies ergibt sich durch die veränderten Flusswiderstände in den jeweiligen Richtungen.

Beispiel 3:

Analyse der Frequenzspektra der Drucksignale

Basis der Berechnung sind die Beträge der komplexen Amplituden $|c_n|$ der einzelnen Fourier-Terme zu den unterschiedlichen Frequenzen nach

$$F(t) = \sum_{-\infty}^{\infty} c_n \cdot e^{(i \cdot 2 \cdot \pi \cdot n \cdot f \cdot t)}$$

(i = imaginäre Einheit, n = Zählparameter, $f$ = Frequenz, t = Zeit).

Die Amplitudenbeträge werden auf die Vektorlänge proportional zur Aufnahmedauer des transformierten Signals normiert, um eine Vergleichbarkeit herzustellen.

Die Frequenzspektren weisen einen regelmäßigen Verlauf auf und zeigen Amplitudenmaxima bei der Grundfrequenz und ganzzahligen Vielfachen davon. Die Frequenzen sind abhängig vom Blutfluss. Eine Verdoppelung des Blutflusses von 100 ml/min auf 200 ml/min führt beispielsweise zu einer Verdoppelung der Grundfrequenz von etwa 0,27 Hz auf 0,54 Hz. Der Amplitudenbetrag $|c_n|$ ist für die blutseitigen Drücke PB1 und PB2 mit sinkender Permeabilität größer und für die dialysatseitigen (PD1 und PD2) verhält es sich umgekehrt.

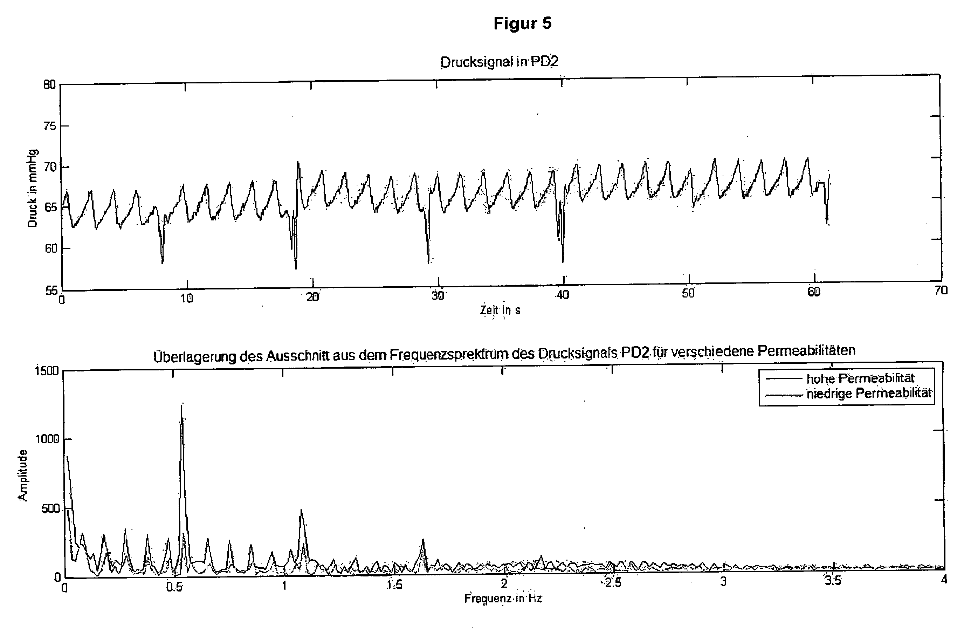

In Figur 4 ist ersichtlich, wie sich die in Beispiel 2 beschriebene Permeabilitätsänderung auf das Frequenzspektrum des Drucksignals PB1 auswirkt. Dies ist in analoger Weise in Figur 5 für PD2 dargestellt.

**Beispiel 4:**

Detektion einer Störung in Form einer Verengung im Schlauchsystem zwischen [PB1] und dem Filter in einem Blutbehandlungssystem

Nach Beginn der Behandlung mit einer Dialysevorrichtung können nach 5 min Vorlaufzeit an den Drucksensoren [PB1], [PB2], [PD1] und [PD2] die Folgenden Drucksignale PB1 = 148 mmHg, PB2 = 61 mmHg, PD1 = 90 mmHg und PD2 = 83 mmHg gemessen werden. Die Messung erfolgt mit Drucksensoren, die eine Abtastrate von 20 Hz haben.

Nach 30 min steigt das Drucksignal am [PB1] plötzlich innerhalb von Sekunden auf 207 mmHg, während alle anderen Drucksignale konstant bleiben. Das schlägt sich weiterhin in einer Steigerung des Transmembrandrucks (TMP) von 18 auf 48 nieder. Dies könnte der Fachmann als deutliche Verschlechterung der Filterpermeabilität, z.B. einer Sekundärmembranbildung fehldeuten, weil er sich auf den TMP verlässt.

Die Betrachtung der Gesamtheit der gemessenen Drucksignale und der Quotienten und/oder der Differenzen der zeitlichen Trends zeigt hingegen, dass alle Drucksensoren einen unveränderten Trend der gemessenen Drucksignale zeigen, außer der Drucksensor [PB1], welcher einen schnell ansteigenden Trend von 148 mmHg auf 207 mmHg aufweist. Die weitere Berechnung der Quotienten und der Differenzen der gemessenen Drucksignale und der Erstellung der zeitlichen Trends zeigt in diesem Fall, dass die zeitlichen Trends der Quotienten der gemessenen Drucksignale (PB1, PB2), (PB1, PD1), (PB1, PD2), (PB1, PDM), (PBM, PD1), (PBM, PD2) und (PBM, PDM) schnell ansteigen, wohingegen die zeitlichen Trends der Quotienten der gemessenen Drucksignale (PB2, PD1), (PB2, PD2), (PB2, PDM) und (PD1, PD2) unverändert geblieben sind.

Aus diesem Muster, umfassend die Trends der gemessenen Drucksignale und die Trends der Quotienten und/oder der Differenzen der gemessenen Drucksignale, wird sofort klar, dass die Störung der Filterpermeabilität nicht die Ursache für die Störung sein kann, sondern, dass eine Verengung im Schlauchsystem zwischen [PB1] und dem Filter aufgetreten sein muss. Eine Störung der Filterpermeabilität kann ausgeschlossen werden, da ansonsten auch die Trends der Quotienten der gemessenen Drucksignale aus (PB2, PD1), (PB2, PD2) und (PB2, PDM) hätten ansteigen müssen und es zusätzlich insgesamt zu einem schnelleren Anstieg aller Trends gekommen wäre. Dies ist in der vorliegenden Störung aber nicht der Fall, womit eine Störung in Form einer Sekundärmembranbildung ausgeschlossen werden kann. Somit wird eine Fehlinterpretation der Störung vermieden und die Störung korrekt lokal detektiert und qualitativ erfasst.

**Beispiel 5:**

Detektion einer Störung in Form einer Verengung in der venösen Nadel

Nach Beginn der Behandlung mit einer Dialysevorrichtung können nach 5 min Vorlaufzeit an den Drucksensoren [PB1], [PB2], [PD1] und [PD2] die Folgenden Drucksignale PB1 = 155 mmHg, PB2 = 68 mmHg, PD1 = 88 mmHg und PD2 = 77 mmHg gemessen werden. Die Messung erfolgt mit Drucksensoren, die eine Abtastrate von 20 Hz haben.

Während der Behandlung steigen die Drucksignale auf der Blutseite PB1 von 155 mmHg auf 178 mmHg und PB2 von 68 mmHg auf 91 mmHg. Auf der Dialysatseite steigen die Drucksignale ebenfalls. PD1 von 88 mmHg auf 111 mmHg und PD2 von 77 mmHg auf 100 mmHg.

Die weitere Berechnung der Quotienten und der Differenzen der gemessenen Drucksignale und der Erstellung der zeitlichen Trends zeigt in diesem Fall, dass die zeitlichen Trends der Quotienten aller Drucksignale einen abfallenden Trend aufweisen, wohingegen die zeitlichen Trends der Differenzen aller Drucksignale einen unverändert gebliebenen Trend aufweisen. Dieses Störungsbild entspricht einer Verengung in der venösen Nadel und wird erfindungsgemäß detektiert.

Bei einer Verengung in der venösen Nadel verändern sich die Filtereigenschaften nicht, aber die Dialysemaschine passt die dialysatseitigen Drücke dennoch um den gleichen Wert an, da der Ultrafiltrationsfluss konstant gehalten werden muss. Der TMP bleibt ebenfalls konstant. Genauso alle weiteren Verhältnisse, die sich aus der Differenzbildung ergeben, da alle Werte sich absolut betrachtet gleich ändern. Da aber die relativen Änderungen für die jeweiligen Drucksignale unterschiedlich groß sind, steigen die Quotienten, welche aus den Drucksignalen gebildet werden. Dies wird besonders deutlich bei Betrachtung des TMP (PBM - PDM) und der Quotienten aus blut- und dialysatseitigem Druck.

TMP(vorher) = Die Differenz aus PBM - PDM = 29 mmHg
TMP (nachher) = Die Differenz aus PBM - PDM = 29 mmHg
Der Quotient aus PBM(vorher) / PDM (vorher) = 1,35
Der Quotient aus PBM(nachher) / PDM (nachher) = 1,27

Durch Beobachtung der Trends der Quotienten und der Differenzen wird die Verengung in der venösen Nadel damit frühzeitig als Verstopfung des Rücklaufs des Blutes zum Patienten erkannt, womit einer drohenden kritischen Situation durch einen zu stark verschlossenen Rücklauf vorgebeugt werden kann, welche ansonsten unbemerkt geblieben wäre.

**Referenzzeichenliste:**

PB1      Druck gemessen am Drucksensor (PB1)
PB2      Druck gemessen am Drucksensor (PB2)
PD1      Druck gemessen am Drucksensor (PD1)
PD2      Druck gemessen am Drucksensor (PD2)
[PB1, PB2, PD1, PD2]:      Drucksensoren
[PB1]      Blutseitiger Drucksensor vor dem Tangentialflussfilter
[PB2]      Blutseitiger Drucksensor hinter dem Tangentialflussfilter
[PD1]      Dialysatseitiger Drucksensor vor dem Tangentialflussfilter
[PD2]      Dialysatseitiger Drucksensor hinter dem Tangentialflussfilter
$A_{PB1}$      Amplitude des am Drucksensor (PB1) gemessenen Drucks PB1
$A_{PB2}$      Amplitude des am Drucksensor (PB2) gemessenen Drucks PB2
$A_{PD1}$      Amplitude des am Drucksensor (PD1) gemessenen Drucks PD1
$A_{PD2}$      Amplitude des am Drucksensor (PD2) gemessenen Drucks PD2
UFP      Ultrafiltrationspumpe
P      Pumpe
TFF      Tangentialflussfilter
☺      Patient
BK      Bilanzkammer

**Patentansprüche**

1. Verfahren zur Unterscheidung von Störungen des Flusswiderstandes in einem Blutbehandlungssystem, das die folgenden Schritte umfasst:

a) Messung von mindestens drei Drucksignalen ausgewählt aus der Gruppe bestehend aus (PB1, PB2, PD1, PD2), die an mindestens drei Drucksensoren gemessen werden, die aus der Gruppe bestehend aus [PB1], [PB2], [PD1] und [PD2] ausgewählt werden, wobei [PB1] den Drucksensor im Blutkreislauf vor dem Bluteinlass in den Tangentialflussfilter TFF bezeichnet und PB1 den am Drucksensor [PB1] gemessenen Druck, [PB2] den Drucksensor im Blutkreislauf nach dem Blutauslass aus dem Tangentialflussfilter TFF bezeichnet und PB2 den am Drucksensor [PB2] gemessenen Druck, [PD1] den Drucksensor im Dialysatkreislauf vor dem Dialysateinlass in den Tangentialflussfilter TFF bezeichnet und PD1 den am Drucksensor [PD1] gemessenen Druck und [PD2] den Drucksensor im Dialysatkreislauf nach dem Dialysatauslass aus dem Tangentialflussfilter TFF bezeichnet und PD2 den am Drucksensor [PD2] gemessenen Druck;
b) Berechnung der Quotienten und/oder der Differenzen aus den gemäß Schritt a) gemessenen Drucksignalen, wobei die Quotienten und/oder Differenzen berechnet werden aus den Drucksignalen ausgewählt aus der Gruppe bestehend aus (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM) in einer zentralen Recheneinheit, wobei mit PBM oder PDM der jeweilige Mittelwert aus (PB1, PB2) bzw. (PD1, PD2) bezeichnet wird;
c) Mindestens einmaliges zeitversetztes Wiederholen der Schritte a) und b), wobei an denselben mindestens drei Drucksensoren wie in der vorangegangenen Messung gemessen wird und aus den gemessenen Drucksignalen dieselben Quotienten und/oder Differenzen berechnet werden;

d) Erstellen mindestens eines zeitlichen Trends aus den gemessenen Drucksignalen ausgewählt aus der Gruppe bestehend aus (PB1, PB2, PD1, PD2) und/oder den berechneten Differenzen oder Quotienten der Drucksignale (PB1, PB2) und/oder (PB1, PD1) und/oder (PB1, PD2) und/oder (PB2, PD1) und/oder (PB2, PD2) und/oder (PD1, PD2) und/oder (PB1, PDM) und/oder (PB2, PDM) und/oder (PD1, PBM) und/oder (PD2, PBM) und/oder (PDM, PBM);

e) Bewerten des mindestens einen zeitlichen Trends, ob eine Änderung des mindestens einen zeitlichen Trends über einen Toleranzbereich hinaus eingetreten ist;

f) Erstellen eines Musters für die Bewertung des mindestens einen zeitlichen Trends;

g) Zuordnung des Musters zu einem Störungsbild und

h) Anzeigen der Störung auf einer Anzeigevorrichtung der zentralen Recheneinheit.

2. Verfahren gemäß Anspruch 1, wobei die mindestens drei Drucksignale jeweils Absolutdrücke, relative Drücke, absolute Druckdifferenzen zwischen zwei Druckmesspunkten, relative Druckdifferenzen zwischen zwei Druckmesspunkten, absoluten Druckamplituden, relativen Druckamplituden, Differenzen zwischen den absoluten Druckamplituden an zwei Druckmesspunkten oder Differenzen zwischen den relativen Druckamplituden an zwei Druckmesspunkten oder eine Kombination davon sein können.

3. Verfahren gemäß Anspruch 1, wobei die mindestens drei Drucksignale jeweils aus der Analyse des Frequenzspektrums des Blutflusses ermittelt werden.

4. Verfahren zur Unterscheidung von Störungen des Flusswiderstandes in einem Blutbehandlungssystem, das die folgenden Schritte umfasst:

a) Zeitversetzte Messung von jeweils mindestens drei Drucksignalen (PB1, PB2, PD1 oder PD2) an mindestens drei Drucksensoren ([PB1], [PB2], und [PD1] oder [PD2]), wobei

[PB1] den Drucksensor im Blutkreislauf vor dem Bluteinlass in den Tangentialflussfilter TFF und PB1 das an [PB1] gemessene Drucksignal bezeichnet,

[PB2] den Drucksensor im Blutkreislauf nach dem Blutauslass aus dem Tangentialflussfilter TFF und PB2 das an [PB2] gemessene Drucksignal bezeichnet,

[PD1] den Drucksensor im Dialysatkreislauf vor dem Dialysateinlass in den Tangentialflussfilter TFF und PD1 das an [PD1] gemessene Drucksignal bezeichnet, und

[PD2] den Drucksensor im Dialysatkreislauf nach dem Dialysatauslass aus dem Tangentialflussfilter TFF und PD2 das an [PD2] gemessene Drucksignal bezeichnet;

b) Berechnung von mindestens zwei Quotienten und/oder Differenzen aus den gemäß Schritt a) an demselben Drucksensor gemessen zeitversetzten Drucksignalen in einer zentralen Recheneinheit, wobei die derart berechneten Quotienten gemäß dem Drucksensor, an dem die Messung durchgeführt wurde, als Q[PB1], Q[PB2], Q[PD1] und Q[PD2] und die Differenzen als Δ[PB1], Δ[PB2], Δ[PD1] und Δ[PD2] bezeichnet werden;

c) Berechnung mindestens eines Quotienten und/oder mindestens einer Differenz in der zentralen Recheneinheit aus den gemäß Schritt b) berechneten Quotienten und/oder Differenzen, wobei die Quotienten und Differenzen aus der Gruppe bestehend aus (Q[PB1], Q[PB2]) und/oder (Q[PB1], Q[PD1]) und/oder (Q[PB1], Q[PD2]) und/oder (Q[PB2], Q[PD1]) und/oder (Q[PB2], Q[PD2]) und/oder (Q[PD1], Q[PD2]) und/oder (Δ[PB1], Δ[PB2]) und/oder (Δ[PB1], Δ[PD1]) und/oder (Δ[PB1], Δ[PD2]) und/oder (Δ[PB2], Δ[PD1]) und/oder (Δ[PB2], Δ[PD2]) und/oder (Δ[PD1], Δ[PD2]) und/oder (Q[PB1], (Δ[PB1]) und/oder (Q[PB1], (Δ[PB2]) und/oder (Q[PB1], (Δ[PD1]) und/oder (Q[PB1], (Δ[PD2]) und/oder (Q[PB2], (Δ[PB1]) und/oder (Q[PB2], (Δ[PB2]) und/oder (Q[PB2], (Δ[PD1]) und/oder (Q[PB2], (Δ[PD2]) und/oder (Q[PD1], (Δ[PB1]) und/oder (Q[PD1], (Δ[PB2]) und/oder (Q[PD1], (Δ[PD1]) und/oder (Q[PD1], (Δ[PD2]) und/oder (Q[PD2], (Δ[PB1]) und/oder (Q[PD2], (Δ[PB2]) und/oder (Q[PD2], (Δ[PD1]) und/oder (Q[PD2], (Δ[PD2])

und/oder (Q[PB1], Q[PBM]) und/oder (Q[PB2], Q[PBM]) und/oder (Q[PD1], Q[PBM]) und/oder (Q[PD2], Q[PBM]) und/oder (Δ[PB1], Q[PBM]) und/oder (Δ[PB2], Q[PBM]) und/oder (Δ[PD1], Q[PBM]) und/oder (Δ[PD2], Q[PBM]) und/oder (Q[PB1], Q[PDM]) und/oder (Q[PB2], Q[PDM]) und/oder (Q[PD1], Q[PDM]) und/oder (Q[PD2], Q[PDM]) und/oder (Δ[PB1], Q[PDM]) und/oder (Δ[PB2], Q[PDM]) und/oder (Δ[PD1], Q[PDM]) und/oder (Δ[PD2], Q[PDM]) und/oder (Q[PB1], Δ[PBM]) und/oder (Q[PB2], Δ[PBM]) und/oder (Q[PD1], Δ[PBM]) und/oder (Q[PD2], Δ[PBM]) und/oder (Δ[PB1], Δ[PBM]) und/oder (Δ[PB2], Δ[PBM]) und/oder (Δ[PD1], Δ[PBM]) und/oder (Δ[PD2], Δ[PBM]) und/oder (Q[PB1], Δ[PDM]) und/oder (Q[PB2], Δ[PDM]) und/oder (Q[PD1], Δ[PDM]) und/oder (Q[PD2], Δ[PDM]) und/oder (Δ[PB1], Δ[PDM]) und/oder (Δ[PB2], Δ[PDM]) und/oder (Δ[PD1], Δ[PDM]) und/oder (Δ[PD2], Δ[PDM]) und/oder (Q[PBM], Q[PDM]) und/oder (Q[PBM], Δ[PBM]) und/oder (Q[PBM], Δ[PDM]) und/oder (Q[PDM], Δ[PBM]) und/oder (Q[PDM], Δ[PDM]) und/oder (Δ[PBM], Δ[PDM]) ausgewählt werden und wobei Q[PBM] den Mittelwert aus Q[PB1] und Q[PB2], Q[PDM] den Mittelwert aus Q[PD1] und Q[PD2], Δ[PBM] den Mittelwert aus Δ[PB1] und Δ[PB2] bezeichnet, Δ[PDM] den Mittelwert aus Δ[PD1] und Δ[PD2] bezeichnet;

d) Mindestens einmaliges zeitversetztes Wiederholen der Schritte a), b) und c), wobei an denselben mindestens drei Drucksensoren wie in der vorangegangenen Messung gemessen wird und aus den gemessenen Drucksignalen dieselben Quotienten und/oder Differenzen berechnet werden;

e) Erstellen mindestens eines zeitlichen Trends aus den gemessenen Drucksignalen ausgewählt aus den in c) gelisteten Gruppen;

f) Bewerten des mindestens einen zeitlichen Trends, ob eine Änderung des mindestens einen zeitlichen Trends über einen Toleranzbereich hinaus eingetreten ist;

g) Erstellen eines Musters für die Bewertung des mindestens einen zeitlichen Trends;

h) Zuordnung des Musters zu einem Störungsbild und

i) Anzeigen der Störung auf einer Anzeigevorrichtung der zentralen Recheneinheit.

5. Verfahren gemäß Anspruch 4, wobei die mindestens drei Drucksignale jeweils Absolutdrücke, relative Drücke, absolute Druckdifferenzen zwischen zwei Druckmesspunkten, relative Druckdifferenzen zwischen zwei Druckmesspunkten, absolute Druckamplituden, relative Druckamplituden, eine Differenz zwischen den absoluten Druckamplituden an zwei Druckmesspunkten oder eine Differenz zwischen den relativen Druckamplituden an zwei Druckmesspunkten oder eine Kombination davon sind.

6. Verfahren gemäß Anspruch 4 oder 5, wobei die mindestens drei Drucksignale aus der Analyse des Frequenzspektrums des Blutflusses ermittelt wird.

7. Ein Blutbehandlungssystem, das aus einer Blutbehandlungseinheit und mindestens drei Drucksensoren [PB1], [PB2], und einem Drucksensor ausgewählt aus [PD1] und [PD2], wobei [PB1] den Drucksensor im Blutkreislauf vor dem Bluteinlass in den Tangentialflussfilters TFF, [PB2] den Drucksensor im Blutkreislauf nach dem Blutauslass aus dem Tangentialflussfilter TFF, [PD1] den Drucksensor im Dialysatkreislauf vor dem Dialysateinlass in den Tangentialflussfilter TFF und [PD2] den Drucksensor im Dialysatkreislauf nach dem Dialysatauslass aus dem Tangentialflussfilter TFF bezeichnet und einer zentralen Recheneinheit besteht, wobei die mindestens drei Drucksensoren mit der zentralen Recheneinheit zur Übertragung der Messwerte verbunden sind und die zentrale Recheneinheit befähigt ist, die eingehenden Messwerte derart auszuwerten und anzuzeigen, daß eine Unterscheidung von Flusswiderstandsänderungen in transmembraner Richtung und Blutflussrichtung in der Blutbehandlungseinheit nach einem der Verfahren gemäß einem der Ansprüche 1 bis 6 möglich ist.

8. Das Blutbehandlungssystem gemäß Anspruch 7, das zusätzlich eine Ultrafiltrationspumpe und ein Bilanzkammersystem umfasst.

9. Das Blutbehandlungssystem gemäß Anspruch 7 oder 8, bei dem die Drucksensoren [PB1], [PB2], [PD1] und [PD2] jeweils eine Abtastrate von mindestens 20 Hz haben.

**Claims**

1. Method for distinguishing disruptions of the flow resistance in a blood treatment system which comprises the following steps:

a) measuring at least three pressure signals selected from the group comprising (PB1, PB2, PD1, PD2) which are measured on at least three pressure sensors which are selected from the group comprising [PB1], [PB2], [PD1] and [PD2], wherein [PB1] refers to the pressure sensor in the blood circulation upstream of the blood inlet in the tangential flow filter TFF and PB1 refers to the pressure measured at the pressure sensor [PB1], [PB2] refers to the pressure sensor in the blood circulation downstream of the blood outlet from the tangential flow filter TFF and PB2 refers to the pressure measured at the pressure sensor [PB2], [PD1] refers to the pressure sensor in the dialysate circuit upstream of the dialysate inlet in the tangential flow filter TFF and PD1 refers to the pressure measured at the pressure sensor [PD1] and [PD2] refers to the pressure sensor in the dialysate circuit downstream of the dialysate outlet from the tangential flow filter TFF and PD2 refers to the pressure measured at the pressure sensor [PD2];

b) calculating the quotients and/or the differences from the pressure signals measured according to step a), wherein the quotients and/or differences are calculated from the pressure signals selected from the group comprising (PB1, PB2) and/or (PB1, PD1) and/or (PB1, PD2) and/or (PB2, PD1) and/or (PB2, PD2) and/or (PD1, PD2) and/or (PB1, PDM) and/or (PB2/PDM) and/or (PD1, PBM) and/or (PD2, PBM) and/or (PDM, PBM) in a central processing unit, wherein PBM or PDM refers to the respective mean value from (PB1, PB2) or (PD1, PD2);

c) at least one occurrence of time-delayed repetition of the steps a) and b), wherein a measurement operation is carried out at the same at least three pressure sensors as in the previous measurement and from the measured pressure signals the same quotients and/or differences are calculated;

d) producing at least one time trend from the measured pressure signals selected from the group comprising (PB1, PB2, PD1, PD2) and/or the calculated differences or quotients of the pressure signals (PB1, PB2) and/or (PB1, PD1) and/or (PB1, PD2) and/or (PB2, PD1) and/or (PB2, PD2) and/or (PD1, PD2) and/or (PB1, PDM) and/or (PB2, PDM) and/or (PD1, PBM) and/or (PD2, PBM) and/or (PDM, PBM);

e) evaluating the at least one time trend, whether a change of the at least one time trend beyond a tolerance range has occurred;

f) producing a pattern for the evaluation of the at least one time trend;

g) associating the pattern with an interference image and

h) indicating the interference on a display device of the central processing unit.

2. Method according to claim 1, wherein the at least three pressure signals may each be absolute pressures, relative pressures, absolute pressure differences between two pressure measurement locations, relative pressure differences between two pressure measurement locations, absolute pressure amplitudes, relative pressure amplitudes, differences between the absolute pressure amplitudes at two pressure measurement locations or differences between the relative pressure amplitudes at two pressure measurement locations or a combination thereof.

3. Method according to claim 1, wherein the at least three pressure signals are each established from the analysis of the frequency spectrum of the blood flow.

4. Method for distinguishing disruptions of the flow resistance in a blood treatment system which comprises the following steps:

a) time-delayed measurement of at least three pressure signals (PB1, PB2, PD1 or PD2) on at least three pressure sensors ([PB1], [PB2] and [PD1] or [PD2]), wherein
[PB1] refers to the pressure sensor in the blood circulation upstream of the blood inlet in the tangential flow filter TFF and PB1 refers to the pressure signal measured at [PB1], [PB2] refers to the pressure sensor in the blood circulation downstream of the blood outlet from the tangential flow filter TFF and PB2 refers to the pressure signal measured at [PB2],
[PD1] refers to the pressure sensor in the dialysate circuit upstream of the dialysate inlet in the tangential flow filter TFF and PD1 refers to the pressure signal measured at [PD1], and
[PD2] refers to the pressure sensor in the dialysate circuit downstream of the dialysate outlet from the tangential flow filter TFF and PD2 refers to the pressure signal measured at [PD2];

b) calculating at least two quotients and/or differences from the time-delayed pressure signals measured according to step a) on the same pressure sensor in a central processing unit, wherein the quotients calculated in this manner, in accordance with the pressure sensor on which the measurement was carried out, are referred to as Q[PB1], Q[PB2], Q[PD1] and Q[PD2] and the differences are referred to as $\Delta$[PB1], $\Delta$[PB2], $\Delta$[PD1] and $\Delta$[PD2];

c) calculating at least one quotient and/or at least one difference in the central processing unit from the quotients and/or differences calculated according to step b),
wherein the quotients and differences are selected from the group comprising (Q[PB1], Q[PB2]) and/or (Q[PB1], Q[PD1]) and/or (Q[PB1], Q[PD2]) and/or (Q[PB2], Q[PD1]) and/or (Q[PB2], Q[PD2]) and/or (Q[PD1], Q[PD2]) and/or ($\Delta$[PB1], $\Delta$[PB2]) and/or ($\Delta$[PB1], ($\Delta$[PD1]) and/or ($\Delta$[PB1], $\Delta$[PD2]) and/or ($\Delta$[PB2], $\Delta$[PD1]) and/or ($\Delta$[PB2], $\Delta$[PD2]) and/or ($\Delta$[PD1], $\Delta$[PD2]) and/or (Q[PB1], ($\Delta$[PB1]) and/or (Q[PB1], $\Delta$[PB2]) and/or (Q[PB1], ($\Delta$[PD1]) and/or (Q[PB1], ($\Delta$[PD2]) and/or (Q[PB2], $\Delta$[PB1]) and/or (Q[PB2], ($\Delta$[PB2]) and/or (Q[PB2], ($\Delta$[PD1]) and/or (Q[PB2], ($\Delta$[PD2]) and/or (Q[PD1], ($\Delta$[PB1]) and/or (Q[PD1], ($\Delta$[PB2]) and/or (Q[PD1], ($\Delta$[PD1]) and/or (Q[PD1], ($\Delta$[PD2]) and/or (Q[PD2], ($\Delta$[PB1]) and/or (Q[PD2], ($\Delta$[PB2]) and/or (Q[PD2], ($\Delta$[PD1]) and/or (Q[PD2], ($\Delta$[PD2]))

and/or (Q[PB1], Q[PBM]) and/or (Q[PB2], Q[PBM]) and/or (Q[PD1], Q[PBM]) and/or (Q[PD2], Q[PBM]) and/or ($\Delta$[PB1], Q[PBM]) and/or ($\Delta$[PB2], Q[PBM]) and/or ($\Delta$[PD1], Q[PBM]) and/or ($\Delta$[PD2], Q[PBM]) and/or (Q[PB1], Q[PDM]) and/or (Q[PB2], Q[PDM]) and/or (Q[PD1], Q[PDM]) and/or (Q[PD2], Q[PDM]) and/or (Δ[PB1], Q[PDM]) and/or (Δ[PB2], Q[PDM]) and/or (Δ[PD1], Q[PDM]) and/or (Δ[PD2], Q[PDM]) and/or (Q[PB1], Δ[PBM]) and/or (Q[PB2], Δ[PBM]) and/or (Q[PD1], Δ[PBM]) and/or (Q[PD2], Δ[PBM]) and/or (Δ[PB1], Δ[PBM]) and/or (Δ[PB2], Δ[PBM]) and/or (Δ[PD1], Δ[PBM]) and/or (Δ[PD2], Δ[PBM])),

and/or (Q[PB1], Δ[PDM])) and/or (Q[PB2], Δ[PDM]) and/or (Q[PD1], Δ[PDM]) and/or (Q[PD2], Δ[PDM]) and/or (Δ[PB1], Δ[PDM]) and/or (Δ[PB2], Δ[PDM]) and/or (Δ[PD1], Δ[PDM]) and/or (Δ[PD2], Δ[PDM]) and/or (Q[PBM], Q[PDM]) and/or (Q[PBM], Δ[PBM]) and/or (Q[PBM], Δ[PDM]) and/or Q[PDM], Δ[PBM]] and/or (Q[PDM], Δ[PDM] and/or (Δ[PBM], Δ[PDM]), and

wherein Q[PBM] refers to the mean value of Q[PB1] and Q[PB2], Q[PDM] refers to the mean value of Q[PD1] and Q[PD2], Δ[PBM] refers to the mean value of Δ[PB1] and Δ[PB2], Δ[PDM] refers to the mean value of Δ[PD1] and Δ[PD2];

d) at least one occurrence of time-delayed repetition of the steps a), b) and c), wherein a measurement operation is carried out on the same at least three pressure sensors as in the previous measurement and from the measured pressure signals the same quotients and/or differences are calculated;

e) producing at least one time trend from the measured pressure signals selected from the groups listed in c);

f) evaluating the at least one time trend, whether a change of the at least one time trend beyond a tolerance range has occurred;

g) producing a pattern for the evaluation of the at least one time trend;

h) associating the pattern with an interference image and

i) indicating the interference on a display device of the central processing unit.

5. Method according to claim 4, wherein the at least three pressure signals are absolute pressures, relative pressures, absolute pressure differences between two pressure measurement locations, relative pressure differences between two pressure measurement locations, absolute pressure amplitudes, relative pressure amplitudes, a difference between the absolute pressure amplitudes at two pressure measurement locations or a difference between the relative pressure amplitudes at two pressure measurement locations or a combination thereof, respectively.

6. Method according to claim 4 or claim 5, wherein the at least three pressure signals are established from the analysis of the frequency spectrum of the blood flow.

7. A blood treatment system which comprises a blood treatment unit and at least three pressure sensors [PB1], [PB2], and a pressure sensor selected from [PD1] and [PD2], wherein [PB1] refers to the pressure sensor in the blood circulation upstream of the blood inlet in the tangential flow filter TFF, [PB2] refers to the pressure sensor in the blood circulation downstream of the blood outlet from the tangential flow filter TFF, [PD1] refers to the pressure sensor in the dialysate circuit upstream of the dialysate inlet in the tangential flow filter TFF and [PD2] refers to the pressure sensor in the dialysate circuit downstream of the dialysate outlet from the tangential flow filter TFF, and a central processing unit, wherein the at least three pressure sensors are connected to the central processing unit in order to transmit the measurement values and the central processing unit is capable of evaluating and displaying the incoming measurement values in such a manner that a differentiation of fluid resistance changes in the transmembrane direction and blood flow direction is possible in the blood treatment unit in accordance with one of the methods according to any one of claims 1 to 6.

8. The blood treatment system according to claim 7 which further comprises an ultrafiltration pump and a balance chamber system.

9. The blood treatment system according to claim 7 or claim 8, wherein the pressure sensors [PB1, [PB2], [PD1] and [PD2] each have a sampling rate of at least 20 Hz.

**Revendications**

1. Procédé pour différencier des dysfonctionnements de la résistance à l'écoulement dans un système de traitement du sang, lequel procédé comprend les étapes suivantes :

a) la mesure d'au moins trois signaux de pression choisis parmi le groupe constitué de (PB1, PB2, PD1, PD2), qui sont mesurés au niveau au moins de trois capteurs de pression, qui sont choisis parmi le groupe constitué de [PB1], [PB2], [PD1] et [PD2], dans lequel [PB1] désigne le capteur de pression dans le circuit du sang avant l'entrée de sang dans le filtre de flux tangentiel TFF et PB1 désigne la pression mesurée au niveau du capteur de pression [PB1], [PB2] désigne le capteur de pression dans le circuit du sang après la sortie de sang hors du filtre de flux tangentiel TFF et PB2 désigne la pression mesurée au niveau du capteur de pression [PB2], [PD1] désigne le capteur de pression dans le circuit de dialysat avant l'entrée de dialysat dans le filtre de flux tangentiel TFF et PD1 désigne la pression mesurée au niveau du capteur de pression [PD1], et [PD2] désigne le capteur de pression dans le circuit de dialysat après la sortie de dialysat hors du filtre de flux tangentiel TFF et PD2 désigne la pression mesurée au niveau du capteur de pression [PD2] ;

b) le calcul des quotients et/ou des différences à partir des signaux de pression mesurés selon l'étape a), dans lequel les quotients et/ou les différences sont calculés à partir des signaux de pression choisis parmi le groupe constitué de (PB1, PB2), et/ou (PB1, PD1) et/ou (PB1, PD2) et/ou (PB2, PD1) et/ou (PB2, PD2) et/ou (PD1, PD2) et/ou (PB1, PDM) et/ou (PB2, PDM) et/ou (PD1, PBM) et/ou (PD2, PBM) et/ou (PDM, PBM) dans une unité de calcul centrale, dans lequel la valeur moyenne respective résultant de (PB1, PB2) ou (PD1, PD2) est désignée par PBM ou PDM ;

c) la répétition au moins une fois avec un décalage dans le temps des étapes a) et b), dans laquelle la mesure est effectuée au niveau des mêmes au moins trois capteurs de pression comme dans la mesure précédente et que les mêmes quotients et/ou différences sont calculés à partir des signaux de pression mesurés ;

d) l'élaboration au moins d'une tendance dans le temps à partir des signaux de pression mesurés choisis parmi le groupe constitué de (PB1, PB2, PD1, PD2) et/ou des différences ou quotients calculés des signaux de pression (PB1, PB2) et/ou (PB1, PD1) et/ou (PB1, PD2) et/ou (PB2, PD1) et/ou (PB2, PD2) et/ou (PD1, PD2) et/ou (PB1, PDM) et/ou (PB2, PDM) et/ou (PD1, PBM) et/ou (PD2, PBM) et/ou (PDM, PBM) ;

e) l'évaluation de l'au moins une tendance temporelle pour savoir si un changement de l'au moins une tendance temporelle est apparu au-delà d'une plage de tolérance ;

f) l'élaboration d'un modèle pour l'évaluation de l'au moins une tendance temporelle ;

g) l'attribution du modèle à un schéma de dysfonctionnement ; et

h) l'affichage du dysfonctionnement sur un dispositif d'affichage de l'unité de calcul centrale.

2. Procédé selon la revendication 1, dans lequel les au moins trois signaux de pression peuvent être respectivement des pressions absolues, des pressions relatives, des différences de pression absolues entre deux points de mesure de pression, des différences de pression relatives entre deux points de mesure de pression, des amplitudes de pression absolues, des amplitudes de pression relatives, des différences entre les amplitudes de pression absolues au niveau de deux points de mesure de pression ou des différences entre les amplitudes de pression relatives au niveau de deux points de mesure de pression ou une combinaison de ces derniers.

3. Procédé selon la revendication 1, dans lequel les au moins trois signaux de pression sont déterminés respectivement à partir de l'analyse du spectre de fréquence du flux du sang.

4. Procédé pour différencier des dysfonctionnements de la résistance de flux dans un système de traitement du sang, qui comprend les étapes suivantes :

a) la mesure décalée dans le temps de respectivement au moins trois signaux de pression (PB1, PB2, PD1 ou PD2) au niveau au moins de trois capteurs de pression ([PB1], [PB2] et [PD1] ou [PD2]), dans lequel [PB1] désigne le capteur de pression dans le circuit du sang avant l'entrée de sang dans le filtre de flux tangentiel TFF et PB 1 désigne le signal de pression mesuré au niveau de [PB1],

[PB2] désigne le capteur de pression dans le circuit du sang après la sortie de sang hors du filtre de flux tangentiel TFF et PB2 désigne le signal de pression mesuré au niveau de [PB2],

[PD1] désigne le capteur de pression dans le circuit de dialysat avant l'entrée de dialysat dans le filtre de flux tangentiel TFF et PD1 désigne le signal de pression mesuré au niveau de [PD1], et

[PD2] désigne le capteur de pression dans le circuit de dialysat après la sortie de dialysat hors du filtre de flux tangentiel TFF et PD2 désigne le signal de pression mesuré au niveau de [PD2] ;

b) le calcul d'au moins deux quotients et/ou différences à partir des signaux de pression décalés dans le temps mesurés selon l'étape a) au niveau du même capteur de pression dans une unité de calcul centrale, dans lequel les quotients calculés de cette manière sont désignés selon le capteur de pression, au niveau duquel la mesure a été effectuée, par Q[PB1], Q[PB2], Q[PD1] et Q[PD2] et que les différences sont désignées par Δ[PB1], Δ[PB2], Δ[PD1] et Δ[PD2] ;

c) le calcul au moins d'un quotient et/ou au moins d'une différence dans l'unité de calcul centrale à partir des quotients et/ou différences calculés selon l'étape b), dans lequel les quotients et différences sont choisis parmi le groupe constitué de (Q[PB1], Q[PB2]) et/ou (Q[PB1], Q[PD1]) et/ou (Q[PB1], Q[PD2]) et/ou (Q[PB2], Q[PD1]) et/ou (Q[PB2], Q[PD2]) et/ou (Q[PD1], Q[PD2]) et/ou (Δ[PB1], Δ[PB2]) et/ou (Δ[PB1], Δ[PD1]) et/ou (Δ[PB1], Δ[PD2]) et/ou (Δ[PB2], Δ[PD1]) et/ou (Δ[PB2], Δ[PD2]) et/ou (Δ[PD1], Δ[PD2]) et/ou (Q[PB1], (Δ[PB1]) et/ou (Q[PB1], (Δ[PB2]) et/ou (Q[PB1], (Δ[PD1]) et/ou (Q[PB1], (Δ[PD2]) et/ou (Q[PB2], (Δ(PB1)) et/ou (Q[PB2], (Δ[PB2]) et/ou (Q[PB2], (Δ[PD1]) et/ou (Q[PB2], (Δ[PD2]) et/ou (Q[PD1], (Δ[PB1]) et/ou (Q[PD1], (Δ[PB2]) et/ou (Q[PD1], (Δ[PD1]) et/ou (Q[PD1], (Δ[PD2]) et/ou (Q[PD2], (Δ[PB1]) et/ou (Q[PD2], (Δ[PB2]) et/ou (Q[PD2], (Δ[PD1]) et/ou (Q[PD2], (Δ[PD2]) et/ou (Q[PB1], Q[PBM]) et/ou (Q[PB2], Q[PBM]) et/ou (Q[PD1], Q[PBM]) et/ou (Q[PD2], Q[PBM]) et/ou (Δ[PB1], Q[PBM]) et/ou (Δ[PB2], Q[PBM]) et/ou (Δ[PD1], Q[PBM]) et/ou (Δ[PB2], Q[PBM]) et/ou (Q[PB1], Q[PDM]) et/ou (Q[PB2], Q[PDM]) et/ou (Q[PD1], Q[PDM]) et/ou (Q[PD2], Q[PDM]) et/ou (Δ[PB1], Q[PDM]) et/ou (Δ[PB2], Q[PDM]) et/ou (Δ[PD1], Q[PDM]) et/ou (Δ[PD2], Q[PDM]) et/ou (Q[PB1], Δ[PBM]) et/ou (Q[PB2], Δ [PBM]) et/ou (Q[PD1], Δ[PBM]) et/ou (Q[PD2], Δ[PBM]) et/ou (Δ[PB1], Δ[PBM]) et/ou Δ[PB2], Δ[PBM]) et/ou (Δ[PD1], Δ[PBM]) et/ou (Δ[PD2], Δ[PBM]]) et/ou Q[PB1], Δ[PDM]) et/ou Q[PB2], Δ[PDM]) et/ou (Q[PD1], Δ[PDM]) et/ou (Q[PD2], Δ[PDM]) et/ou Δ[PB1], Δ[PDM]) et/ou (Δ[PB2]), Δ[PDM]) et/ou (Δ[PD1]), Δ[PDM]) et/ou (Δ[PD2]), Δ[PDM]) et/ou (Q[PBM], Q[PDM]) et/ou (Q[PBM], Δ[PBM]) et/ou (Q[PBM], Δ[PDM]) et/ou (Q[PDM], Δ[PBM]) et/ou (Q[PDM], Δ[PDM]) et/ou (Δ[PBM], Δ[PDM]), et

dans lequel Q[PBM] désigne la valeur moyenne résultant de Q[PB1] et de Q[PB2], Q[PDM] désigne la valeur moyenne résultant de Q[PD1] et Q[PD2], Δ[PBM] désigne la valeur moyenne résultant de Δ[PB1] et de Δ[PB2], Δ[PDM] désigne la valeur moyenne résultant de Δ[PD1] et de Δ[PD2] ;

d) la répétition au moins une fois avec un décalage dans le temps des étapes a), b) et c), dans laquelle la mesure est effectuée au niveau des mêmes au moins trois capteurs de pression comme dans la mesure précédente et que les mêmes quotients et/ou différences sont calculés à partir des signaux de pression mesurés ;

e) l'élaboration au moins d'une tendance temporelle à partir des signaux de pression mesurés choisis parmi les groupes listés au point c) ;

f) l'évaluation de l'au moins une tendance temporelle pour savoir si un changement de l'au moins une tendance temporelle est apparu au-delà d'une plage de tolérance ;

g) l'élaboration d'un modèle pour l'évaluation de l'au moins une tendance temporelle ;

h) l'attribution du modèle à un schéma de dysfonctionnement ; et

i) l'affichage du dysfonctionnement sur un dispositif d'affichage de l'unité de calcul centrale.

5. Procédé selon la revendication 4, dans lequel les au moins trois signaux de pression sont respectivement des pressions absolues, des pressions relatives, des différences de pression absolues entre deux points de mesure de pression, des différences de pression relatives entre deux points de mesure de pression, des amplitudes de pression absolues, des amplitudes de pression relatives, une différence entre les amplitudes de pression absolues au niveau de deux points de mesure de pression ou une différence entre les amplitudes de pression relatives au niveau de deux points de mesure de pression ou une combinaison de ces derniers.

6. Procédé selon la revendication 4 ou 5, dans lequel les au moins trois signaux de pression sont déterminés à partir de l'analyse du spectre de fréquence du flux du sang.

7. Système de traitement du sang, qui est constitué d'une unité de traitement du sang et au moins de trois capteurs de pression [PB1], [PB2] et d'un capteur de pression choisi parmi [PD1] et [PD2], dans lequel [PB1] désigne le capteur de pression dans le circuit du sang avant l'entrée du sang dans le filtre de flux tangentiel TFF, [PB2] désigne le capteur de pression dans le circuit du sang après la sortie de sang hors du filtre de flux tangentiel TFF, [PD1] désigne le capteur de pression dans le circuit de dialysat avant l'entrée de dialysat dans le filtre de flux tangentiel TFF et [PD2] désigne le capteur de pression dans le circuit de dialysat après la sortie de dialysat hors du filtre de flux tangentiel TFF, et d'une unité de calcul centrale, dans lequel les au moins trois capteurs de pression sont reliés à l'unité de calcul centrale afin de transmettre les valeurs de mesure et que l'unité de calcul centrale est en mesure d'analyser et d'afficher les valeurs de mesure entrantes de telle manière qu'une différenciation des changements de résistance de flux est possible dans le sens transmembranaire et dans le sens de flux du sang dans l'unité de traitement du sang selon l'un des procédés selon l'une quelconque des revendications 1 à 6.

8. Système de traitement du sang selon la revendication 7, qui comprend en supplément une pompe d'ultra filtration et un système de chambre de détermination de bilan.

9. Système de traitement du sang selon la revendication 7 ou 8, dans lequel les capteurs de pression [PB1], [PB2], [PD1] et [PD2] ont respectivement un taux d'échantillonnage d'au moins 20 Hz.

Figur 1

Figur 2 A

Druckverhalten bei hoher Permeabilität und UF=0

Druck in mmHg

200 180 160 140 120 100 80 60 40 20 0

Zeit in s

0 6 13 19 25 31 38 44 50 56

PB2 PB1 PD2

A

Figur 2 B

B Druckverhalten bei hoher Permeabilität und UF>0

PB2    PB1    PD2

EP 2 627 368 B1

**Figur 2 C**

Druckverhalten bei niedriger Permeabilität und UF=0

PB2    PB1    PD2

Figur 2 D

Figur 3

EP 2 627 368 B1

Frequenzermittlung und Phasenverschiebung der Drucksignale

PB1          PB2          PD2

## Figur 4

Drucksignal in PB1

Ausschnitt aus dem Frequenzspektrum des Drucksignals PB1

Überlagerung eines Ausschnitts aus dem Frequenzspektrum des Drucksignals PB1 für verschiedene Permeabilitäten

hohe Permeabilität
niedrige Permeabilität

EP 2 627 368 B1

## Figur 5

Drucksignal in PD2

Überlagerung des Ausschnitt aus dem Frequenzsprektrum des Drucksignals PD2 für verschiedene Permeabilitäten

EP 2 627 368 B1

## Figur 6

| Musterkonstellation der Trends | Trends der gemessenen Drucksignale | | | | Trends der Quotienten der gemessenen Drucksignale | | | | | | | | | | | Störung / Befund |
|---|---|---|---|---|---|---|---|---|---|---|---|---|---|---|---|---|
| | PB1 | PB2 | PD1 | PD2 | PB1/ PB2 | PB1/ PD1 | PB1/ PD2 | PB1/ PDM | PB2/ PD1 | PB2/ PD2 | PB2/ PDM | PBM/ PD1 | PBM/ PD2 | PBM/ PDM | PD1/ PD2 | |
| | - | 0 | 0 | 0 | - | - | - | - | 0 | 0 | 0 | - | - | - | 0 | Clotting wird reduziert |
| | - | - | 0 | 0 | 0 | - | - | - | - | - | - | - | - | - | 0 | Verringerung der Hämokonzentration |
| | -- | -- | -- | -- | ++ | ++ | ++ | ++ | ++ | ++ | ++ | ++ | ++ | ++ | ++ | Akute Flussverengung vor [PD1] |
| | -- | -- | 0 | - | ++ | -- | ++ | -- | -- | ++ | - | -- | ++ | -- | ++ | Akute Flussverengung zwischen [PD1] und Filter |
| | + | 0 | 0 | 0 | + | + | + | + | 0 | 0 | 0 | + | + | + | 0 | Geringere Durchflussfläche für das Blut verursacht durch Clotting |
| | + | + | 0 | 0 | 0 | + | + | + | + | + | + | + | + | + | 0 | Stärkere Hämokonzentration |
| | + | 0 | - | - | + | + | + | + | + | + | + | + | + | + | + | Sekundärmembranbildung |
| | + | + | + | + | - | - | - | - | - | - | - | - | - | - | - | Druckniveau auf Patientenseite steigt (z.B. höherer Eingangshämatokrit, Änderung der Armposition, Flussveränderung im Zugang) |
| | ++ | 0 | 0 | 0 | ++ | ++ | ++ | ++ | 0 | 0 | 0 | ++ | ++ | ++ | 0 | Schlauchsystem zwischen [PB1] und Filter zugesetzt |
| | ++ | 0 | ++ | ++ | ++ | 0 | 0 | 0 | -- | -- | -- | -- | -- | -- | 0 | Schlauchsystem zwischen Filter und [PB2] zugesetzt |
| | ++ | ++ | ++ | ++ | -- | -- | -- | -- | -- | -- | -- | -- | -- | -- | -- | Schlauchsystem hinter [PB2] zugesetzt oder Flussveränderung im Zugang |
| | 0 | 0 | 0 | -- | 0 | 0 | ++ | ++ | 0 | ++ | ++ | 0 | ++ | ++ | ++ | Akute Flussverengung zwischen Filter und [PD2] |

## Figur 7

| Musterkonstellation der Trends | Trends der gemessenen Drucksignale | | | | Trends der Differenzen der gemessenen Drucksignale | | | | | | | | | | | Störung / Befund |
|---|---|---|---|---|---|---|---|---|---|---|---|---|---|---|---|---|
| | PB1 | PB2 | PD1 | PD2 | PB1-PB2 | PB1-PD1 | PB1-PD2 | PB1-PDM | PB2-PD1 | PB2-PD2 | PB2-PDM | PBM-PD1 | PBM-PD2 | PBM-PDM | PD1-PD2 | |
| | - | 0 | 0 | 0 | - | - | - | - | 0 | 0 | 0 | - | - | - | 0 | Clotting wird reduziert |
| | - | - | 0 | 0 | 0 | - | - | - | - | - | - | - | - | - | 0 | Verringerung der Hämokonzentration |
| | − | − | −− | −− | 0 | 0 | 0 | 0 | 0 | 0 | 0 | 0 | 0 | 0 | 0 | Akute Flussverengung vor [PD1] |
| | − | −− | 0 | −− | 0 | − | 0 | − | −− | 0 | −− | −− | 0 | −− | ++ | Akute Flussverengung zwischen [PD1] und Filter |
| | + | 0 | 0 | 0 | + | + | + | + | 0 | 0 | 0 | + | + | + | 0 | Geringere Durchflussfläche für das Blut verursacht durch Clotting |
| | + | + | 0 | 0 | 0 | + | + | + | + | + | + | + | + | + | 0 | Stärkere Hämokonzentration |
| | + | 0 | - | - | + | + | + | + | + | + | + | + | + | + | 0 | Sekundärmembranbildung |
| | + | + | + | + | 0 | 0 | 0 | 0 | 0 | 0 | 0 | 0 | 0 | 0 | 0 | Druckniveau auf Patientenseite steigt (z.B. höherer Eingangshämatokrit, Änderung der Armposition, Flussveränderung im Zugang) |
| | ++ | 0 | 0 | 0 | ++ | ++ | ++ | ++ | 0 | 0 | 0 | ++ | ++ | ++ | 0 | Schlauchsystem zwischen [PB1] und Filter zugesetzt |
| | ++ | 0 | ++ | ++ | ++ | 0 | 0 | 0 | −− | −− | −− | −− | − | −− | 0 | Schlauchsystem zwischen Filter und [PB2] zugesetzt |
| | ++ | ++ | ++ | ++ | 0 | 0 | 0 | 0 | 0 | 0 | 0 | 0 | 0 | 0 | 0 | Schlauchsystem hinter [PB2] zugesetzt oder Flussveränderung im Zugang |
| | 0 | 0 | 0 | −− | 0 | 0 | ++ | ++ | 0 | ++ | ++ | 0 | ++ | ++ | ++ | Akute Flussverengung zwischen Filter und [PD2] |

**EP 2 627 368 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1364666 A
- US 20080215247 A1
- WO 2006011009 A2
- EP 1175917 A1
- US 2006157408 A1
- DE 10355042 B3